# EUROPÄISCHE PATENTSCHRIFT

(11) **EP 1 461 619 B1**
(45) Veröffentlichungstag und Bekanntmachung des Hinweises auf die Patenterteilung: **27.04.2011**
(21) Anmeldenummer: 02793118.7
(22) Anmeldetag: 27.12.2002
(51) Int. Cl.: G01N 33/543, C12Q 1/68, G01N 33/552, G01N 33/553, B01J 19/00

(54) **VERBESSERTE STRUKTURIERT-FUNKTIONALE BINDEMATRICES FÜR BIOMOLEKÜLE**
IMPROVED STRUCTURED-FUNCTIONAL BONDING MATRICES FOR BIOMOLECULES
MATRICE DE LIAISON A STRUCTURE FONCTIONNELLE AMELIOREE POUR BIOMOLECULES

(30) Priorität: 28.12.2001 DE 10164309
(43) Veröffentlichungstag der Anmeldung: 29.09.2004
(73) Patentinhaber: Fraunhofer-Gesellschaft zur Förderung der angewandten Forschung e.V., 80686 München (DE)
(72) Erfinder: WEBER, Achim, 73776 Altbach (DE); SCHIESTEL, Thomas, 70569 Stuttgart (DE); TOVAR, Günter, 70469 Stuttgart (DE); BRUNNER, Herwig, 70569 Stuttgart (DE); RUPP, Steffen, 70195 Stuttgart (DE); HAUSER, Nicole, 69115 Heidelberg (DE)
(86) Internationale Anmeldenummer: PCT/EP2002/014769
(87) Internationale Veröffentlichungsnummer: WO 2003/056336

(56) Entgegenhaltungen:
- WO-A-01/18242
- WO-A-01/23459
- WO-A-01/73123
- WO-A-98/10289
- C. M. NIEMEYER, B. CEYHAN, S. GAO, L. CHI, S. PESCHEL, U. SIMON: "Site-selective immobilization of gold nanoparticles functionalized with DNA oligomers" COLLOID POLYMER SCIENCE, Bd. 279, Nr. 1, Januar 2001 (2001-01), Seiten 68-72, XP002266039 SPRINGER VERLAG HEIDELBERG
- VO-DINH T: "Surface-enhanced Raman spectroscopy using metallic nanostructures" TRAC, TRENDS IN ANALYTICAL CHEMISTRY, ANALYTICAL CHEMISTRY. CAMBRIDGE, GB, Bd. 17, Nr. 8-9, 9. August 1998 (1998-08-09), Seiten 557-582, XP004146617 ISSN: 0165-9936
- LIU J-F ET AL: "Fabrication of colloidal gold micro-patterns using photolithographed self-assembled monolayers as templates" THIN SOLID FILMS, ELSEVIER-SEQUOIA S.A. LAUSANNE, CH, Bd. 327-329, 31. August 1998 (1998-08-31), Seiten 176-179, XP004151922 ISSN: 0040-6090
- BETHELL D ET AL: "From monolayers to nanostructural materials: an organic chemist's view of self-asembly" JOURNAL OF ELECTROANALYTICAL CHEMISTRY AND INTERFACIAL ELECTROCHEMISTRY, ELSEVIER, AMSTERDAM, NL, Bd. 409, 1996, Seiten 137-143, XP002964036 ISSN: 0022-0728

## Beschreibung

Die vorliegende Erfindung betrifft Funktionselemente, die auf einem Träger angeordnete, aus biofunktionalisierbaren oder biofunktionalisierten Nanopartikeln bestehende Mikrostrukturen umfassen, Verfahren zur Herstellung dieser Funktionselemente sowie die Verwendung derselben.

Die Untersuchung von biologischen Molekülen wie DNA oder Proteinen spielt in den unterschiedlichsten Bereichen eine immer wichtigere Rolle, beispielsweise in der Umweltanalytik zum Nachweis von Mikroorganismen, in der klinischen Diagnostik zur Identifizierung von Erregern oder zur Bestimmung von Resistenzen gegen Medikamente usw. Unabhängig von der speziellen Anwendung müssen diese Analyseverfahren immer die gleichen Anforderungen erfüllen. Insbesondere sollten sie schnell und kostengünstig durchführbar sein, gleichzeitig aber sehr empfindlich sein und zuverlässig reproduzierbare Ergebnisse liefern.

Einen Meilenstein bei der Analyse von biologisch aktiven Molekülen stellen die sogenannten Biochips dar. Mit Hilfe von auch als DNA-Arrays bezeichneten Gen-Chips kann beispielsweise die Nucleinsäure-Bestimmung in zu untersuchenden Proben wesentlich vereinfacht, beschleunigt, parallelisiert, automatisiert und präzisiert werden. Bei diesen Gen-Chips handelt es sich um miniaturisierte Träger, auf deren Oberfläche Nucleinsäuren bekannter Sequenz in einem geordneten Raster immobilisiert oder synthetisiert sind. Gen-Chips werden vorzugsweise in der klinischen Diagnostik von Infektions-, Krebs- und Erbkrankheiten eingesetzt. Die Effizienz solcher Gen-Chips bei der Analyse von Proben beruht insbesondere darauf, dass nur noch minimalisierte Probenvolumina benötigt werden und die Auswertung mittels hochempfindlicher Messverfahren erfolgen kann. Unter Verwendung dieser Chips lassen sich daher große Probenzahlen parallel untersuchen (High Throughput Screening).

Auch Protein-Arrays sind bekannt, bei denen analog zu den Gen-Chips Proteine oder Peptide auf beispielsweise Kunststoffmembranen in einem geordneten und bekannten Raster angeordnet sind. Derartige Protein-Arrays werden vor allem zur Untersuchung der wechselseitigen Bindung von Proteinen, zum Beispiel Rezeptor-Ligand-Wechselwirkungen, zur Identifizierung intrazellulärer Proteinkomplexe, zur Untersuchung von DNA-Protein- und RNA-Protein-Wechselwirkungen oder zur Analyse von Protein-Antikörper-Interaktionen eingesetzt. Mit Hilfe der Protein-Chip-Technologie konnten bereits zahlreiche Proteinmarker für Krebserkrankungen oder Krankheiten wie die Alzheimer-Demenz identifiziert werden.

An einen Träger gebundene biologische Moleküle spielen auch bei der Entwicklung von Biocomputern eine wichtige Rolle. Mit dem "Biocomputer" ist insbesondere der Ersatz herkömmlicher elektronischer Bauelemente wie Transistoren, Dioden, Schalter usw. durch Bauelemente verbunden, die zumindest teilweise aus organischen Materialien bestehen oder diese enthalten. Die Verwendung biologischer Moleküle als elektronische Bauelemente verfolgt vor allem das Ziel, die Fähigkeiten informationsverarbeitender biologischer Systeme, wie beispielsweise Modell- und Objekterkennung, Reproduktion und Selbstorganisation auszunuten. Herkömmliche Bauelemente auf Silicium-Basis besitzen diese Fähigkeiten nicht. Dazu kommt, dass durch die Verwendung biologischer Moleküle ein Miniaturisierungsgrad elektronischer Bauelemente erreicht werden kann, der sich mit Silicium-Bauelementen nicht annähernd realisieren lässt. Eine Miniaturisierung der Bauelemente ist mit einer erheblichen Verbesserung aller signifikanten Parameter von Chips wie Integrationsdichte, Verlustleistung, Schaltgeschwindigkeit und Kosten um Größenordnungen verbunden. Die Grenzen der Miniaturisierung sind bei Bauelementen auf Silicium-Basis zwar noch nicht erreicht, jedoch bereits absehbar, da mit zunehmendem Miniaturisierungsgrad, insbesondere zunehmender Packungsdichte, Probleme wie Wärmeabführung und unerwünschte elektrische Effekte auftreten.

Ein großer Teil der Forschung in der Bioelektronik konzentriert sich heute auf die Verwendung von Proteinen als Grundstrukturen für molekulare Bauelemente. Proteine weisen eine hohe Variabilität in ihrer Funktion und eine hohe Stabilität in ihrer Struktur auf. Bei der Anwendung von Proteinen werden die folgenden Strategien verfolgt: Untersuchung und Modifizierung der Funktionsmechanismen von Proteinen für Logikoperationen, Ausnutzung der Struktureigenschaften für die Anwendung als Trägermoleküle für andersartige molekulare Bausteine und Nutzung der Struktur- und Funktionseigenschaften von Proteinen zur technologischen Herstellung molekularer Schaltkreise. Derzeit werden insbesondere Photorezeptor-Proteine untersucht, die Licht direkt in ein Signal umwandeln können. Dieser Prozess umfasst die Bildung eines elektrischen Dipols und wird von einer Farbveränderung des Proteins begleitet. Aufgrund diese optoelektrischen Eigenschaften lassen sich solche Proteine als "smart material" einsetzen. Das bislang am besten untersuchte Beispiel ist Bakteriorhodopsin, dessen Verwendung in der optischen Informationsverarbeitung als optischer Speicher angestrebt wird.

Derartige bioelektronische Bauelemente können auch zusammen mit herkömmlichen elektronischen Bauelementen im "gemischten" Computersystem Verwendung finden. Die bioelektronischen Bauelemente können jedoch auch in anderen Technikbereichen eingesetzt werden, beispielsweise in der Medizintechnik. Die Verwendung biologischer Materialien in der medizinischen Mess- und Überwachungstechnik sowie für künstliche Organtransplantate und Prothesen bietet viele entscheidende Vorteile. Beispielsweise wird angestrebt, wirkliche Sehhilfen für erblindete Menschen zu entwickeln. So werden derzeit sogenannte Retina-Chips entwickelt, die als Implantat im menschlichen Auge fungieren sollen.

Bei der Generierung von Sensoren und Screeningsystemen in den verschiedenen Bereichen der Analytik ist die Strukturierung von Schichten auf Trägermaterialien von großer Bedeutung. Für die Beschichtung von Trägern aus Metallen, Polymeren, Glas, Halbleitern oder Keramik mit Self-Assembled-Monoschichten, anderen monomolekularen Belegungen, Lacken, Metallfilmen oder Polymerfilmen sind mehrere Verfahren beschrieben. So ist die Abscheidung von nicht-funktionalisierten Partikeln für optische Anwendungen zur Strukturierung von planaren Oberflächen bekannt (Chen, Jiang, Kimerling und Hammond, Langmuir, 16 (2000), 7825-7834).

Fan et al. (Nature, 405 (2000), 56-60) beschreiben die Erzeugung funktioneller, hierarchisch organisierter Strukturen, wobei die Selbstausrichtung von Silica-Tensid-Systemen mit schnellen Druckverfahren, insbesondere der Stift (pen)-Lithographie, dem Tintenstrahl-Drucken und dem Tauchbeschichten strukturierter SAM-Monolayer kombiniert wird. Das beschriebene Verfahren lässt sich zur Herstellung von Sensor-Arrays und Fluid- oder Photonen-Systemen einsetzen. Wichtig für das beschriebene Verfahren ist die Verwendung stabiler, homogener Tintengemische, die nach Evaporation in einem Self-Assembly-Prozess die gewünschte, organisch modifizierte Silica-Tensid-Mesophase bilden können. Dazu wird ein oligomeres Silica-Sol in Ethanol/Wasser bei einer Hydroniumionen-Konzentration hergestellt, die die Siloxan-Kondensationsrate minimiert und so die Oberflächen-Selbstausrichtung des Silica-Tensid-Systems während des Strukturierungsprozesses ermöglicht. Mit der Tintenflüssigkeit wird auf einer Oberfläche ein Muster erzeugt, wobei die bevorzugte Evaporation von Ethanol die Anreicherung von Wasser, Tensid und Silikaten bewirkt, so dass sich ein komplexer dreidimensionaler Konzentrationsgradient bildet. Bei Überschreiten der kritischen Micellen-Konzentration (c.m.c.) werden Micellen erzeugt, wobei mit fortschreitender Evaporation, insbesondere von Wasser, die kontinuierliche Selbstorganisation der Micellen zu Silica-Tensid-Kristallen an den Flüssigkeits-Dampf-Grenzflächen induziert wird. Hydrophobe, Alkohol-lösliche Moleküle können nach Ethanol-Evaporation in dem Netzwerk der resultierenden Mesophase kompartimentiert werden. Unter Verwendung des Mikropen-Lithographie-Verfahrens (MPL) können so Funktionen enthaltende, geordnete Nanostrukturen erzeugt werden. Die Erzeugung solcher Strukturen unter Verwendung funktionalisierbarer Nanopartikel mittels des Mikropen-Lithographie-Verfahrens ist nicht beschrieben.

Die Beschichtung von Trägern mit biologischen Molekülen, insbesondere die strukturierte Beschichtung, ist jedoch wesentlich komplizierter. Die Immobilisierung von Biomolekülen an einen Träger erfolgt im Allgemeinen durch Adsorption oder kovalente Bindung. Dabei müssen die Einzelmoleküle einerseits in hoher Packungsdichte in definierten Abständen positioniert werden. Andererseits müssen die Moleküle so stabilisiert und so an den Träger gebunden werden, dass ihre biologische Aktivität nicht verändert wird oder verloren geht. So sollte bei der Immobilisierung von Proteinen die für die biologische Aktivität erforderliche dreidimensionale Struktur, beispielsweise die dreidimensionale Struktur am aktiven Zentrum eines Enzyms, nicht verändert werden. Das US-Patent Nr. 5,609,907 beschreibt die Herstellung makroskopischer Metalloberflächen unter Verwendung selbstausrichtender kolloidaler Metallpartikel. Dabei werden mit Streptavidin markierte Au-Kolloide auf biotinylierte SAM (self-assembled monolayer)-Oberflächen unstrukturiert aufgebracht. Aufgrund der negativen Ladung der Kolloidpartikel kommt es zu einer Abstoßung der Partikel untereinander. Auf diese Weise wird eine Aggregation mehrerer Partikel verhindert und die Partikel sind als Einzelpartikel mit gleichmäßigem Abstand zueinander angeordnet. Unter Verwendung dieses Verfahrens können also keine kontinuierlichen Strukturen, die mehrere Partikel umfassen, erzeugt werden.

Die Herstellung adhäsiver Templates, insbesondere die strukturierte Anordnung von mit Biotin funktionalisierten Partikeln auf SAM (self-assembled monolayer)-Oberflächen, ist von Harnett et al. (Harnett, Satyalakshmi und Craighead, Langmuir, 17 (2001), 178-182) beschrieben. Dabei werden SAM-Oberflächen mittels Elektronenstrahl-Lithographie strukturiert und anschließend biotinylierte Polystyrol-Partikel (20 nm bis 20 µm) aufgebracht. Bei dem beschriebenen Verfahren wird ein inerter Monolayer mittels Elektronenstrahl-Lithographie behandelt, wobei Strukturen erzeugt werden. Anschließend werden die behandelten und gesäuberten Strukturen mit einem reaktiven Monolayer, an dem Linkermoleküle oder Polystyrol-Partikel mit endständigen Biotin-Einheiten selektiv gebunden sind, verfüllt. Die verwendeten Partikel sind fluoreszierend, können beispielsweise mit Proteinen funktionalisiert werden und sind insbesondere für Zelladhäsions-Anwendungen geeignet. Der zum Verfüllen (backfilling) der Strukturen verwendete reaktive Monolayer kann anschließend wieder entfernt werden, das heißt der Vorgang des Verfüllens kann rückgängig gemacht werden. Das beschriebene Verfahren nutzt dabei insbesondere die Schnelligkeit der Elektronenstrahl-Lithographie beim Schreiben von Einzellinien bzw. Einzelpunkten aus.

Auch die Erzeugung adhäsiver Templates durch Behandlung von Monolayern mit Amin-Gruppen auf Kohlenstoff-Nanotubes mittels Elektronenstrahl-Lithographie und anschließendes Verfüllen mit einem reaktiven Monolayer ist bekannt (Harnett, Satyalakshmi und Craighead, Appl. Phys. Lett., 76 (2000), 2466-2468). Arbeiten zur Nicht-Elektronenstrahl-Lithographie umfassen die Behandlung von Monolayern mittels "scanned probe"-Lithographie (Sugimura und Nakagiri, J. Vac. Sci. Technol., 15 (B 1997), 1394-1397; Wadu-Mesthrige et al., Langmuir, 15 (1999), 8580-8583), das Verfüllen von photolithographisch behandelten hydrophoben Monolayern mit Aminen zur Erzeugung von DNA-Arrays (Brockman, Frutos und Corn, J. Am. Chem. Soc., 121 (1999), 8044-8051) und das routinemäßige Verfüllen zur Funktionalisierung unbedruckter Bereiche beim Mikrokontaktdruckverfahren.

Während sich Nucleinsäuren wie DNA relativ problemlos an Trägern immobilisieren lassen, ist die Situation im Falle von Proteinen ungleich komplizierter. Insbesondere gibt es derzeit nur wenige Verfahren, mit deren Hilfe eine gerichtete Immobilisierung eines Proteins, das heißt insbesondere unter Erhalt seiner Funktionalität, möglich ist. Bei den meisten bekannten Verfahren werden Proteine primär über unspezifische Wechselwirkungen an einer Oberfläche gebunden, das heißt, eine gerichtete kovalente Bindung erfolgt nicht.

Bekannt ist die Beschichtung von Oberflächen mit Hydrogelen, die unterschiedliche Funktionalitäten, beispielsweise Protein-Funktionen, aufweisen können. Die Beschichtung mit Hydrogelen führt zwar im Vergleich zu planaren Oberflächen zu einer Vergrößerung der Oberfläche, gleichzeitig wird aber die unspezifische Fixierung der Funktionalitäten verstärkt.

Die WO 01/23459A beschreibt mulitplexfähige Oberflächensensoren für das SERS-Verfahren, bestehend aus rezeptorderivatisierten Nanopartikeln. Die WO 98/10289A beschreibt Biosensoren auf Basis von derivatisierten Festphasenträgern.

Der vorliegenden Erfindung liegt das technische Problem zugrunde, Mittel und Verfahren zur Erzeugung von miniaturisierten Trägersystemen mit daran immobilisierten biologischen Molekülen, beispielsweise von Gen-Chips und Protein-Chips, zu entwickeln, bei denen die im Stand der Technik bekannten Nachteile beseitigt sind und bei denen die Biomoleküle insbesondere unter Erhalt ihrer biologischen Aktivität in hoher Packungsdichte an einem Träger immobilisiert sind oder immobilisiert werden können und die zur verwendung in unterschiedlichsten Screening- und Analytiksystemen, beispielsweise in der medizinischen Mess- und überwachungstechnik, und in Biocomputern geeignet sind.

Die vorliegende Erfindung löst das ihr zugrunde liegende technische Problem durch die Bereitstellung eines Funktionselementes, umfassend einen Träger mit einer Oberfläche und mindestens eine auf der Trägeroberfläche angeordnete Mikrostruktur, wobei die Mikrostruktur aus Einzelkomponenten in Form von Nanopartikeln besteht, die die Adressierbarkeit der Mikrostruktur ermöglichende molekülspezifische Erkennungsstellen aufweisen, die ausgewählt sind aus: Proteinen, Nucleinsäuremolekülen, Antikörpern und Komplexen davon sowie molekular geprägten Polymeren und einpolymerisierten Surfmeren, wobei die molekülspezifischen Erkennungsstellen mehrere räumlich fixiert angeordnete erste funktionelle Gruppen aufweisen, woran Moleküle unter Beibehaltung deren biologischer Funktion oder Aktivität an definierten Positionen innerhalb der Moleküle über an die ersten funktionellen Gruppen bindende komplementäre zweite funktionelle Gruppen gerichtet bindbar oder gebunden sind, und wobei zwischen der Trägeroberfläche und der Mikrostruktur mindestens eine Schicht eines Verbindungsmittels zur festen Anhaftung der Nanopartikel angeordnet ist und das Verbindungsmittel ausgewählt ist aus: geladenen Polymeren, Plasmaschichten mit geladenen Gruppen, Plasmaschichten mit chemisch reaktiven Gruppen und Kombinationen davon.

Die vorliegende Erfindung stellt also ein Funktionselement bereit, auf dessen Oberfläche ein oder mehrere Mikrostrukturen angeordnet sind, wobei jede Mikrostruktur aus mehreren Nanopartikeln mit identischen oder nicht-identischen molekülspezifischen Erkennungsstellen besteht. Die Mikrostrukturen der Funktionselemente können Biofunktionen aufweisen beziehungsweise damit versehen werden. Das heißt, die molekülspezifischen Erkennungsstellen der die Mikrostruktur bildenden Nanopartikel können entsprechende Moleküle, insbesondere organische Moleküle mit einer biologischen Funktion oder Aktivität, erkennen und diese binden. Bei diesen Molekülen kann es sich beispielsweise um Nucleinsäuren oder Proteine handeln. An die Moleküle, die an die molekülspezifischen Erkennungsstellen der Nanopartikel gebunden sind, können dann andere Moleküle gebunden werden, beispielsweise zu analysierende Moleküle einer Probe. Im Unterschied zu im Stand der Technik bekannten Systemen, beispielsweise herkömmlichen Gen- oder Protein-Arrays, sieht die vorliegende Erfindung also vor, biologische Moleküle nicht direkt an eine planare Oberfläche zu binden, sondern an Nanopartikeloberflächen zu immobilisieren, die vor oder nach der Immobilisierung zur Bildung einer Mikrostruktur eingesetzt werden.

Die erfindungsgemäßen Funktionselemente, die nanopartikuläre Systeme mit molekülspezifischen Erkennungssequenzen zur Bindung biologischer Moleküle umfassen, bieten gegenüber herkömmlichen Systemen, beispielsweise solchen, bei denen die biologischen Moleküle direkt am Träger immobilisiert sind, mehrere entscheidende Vorteile.

Die erfindungsgemäß eingesetzten Nanopartikel stellen äußerst flexible und inerte Systeme dar. Sie können beispielsweise aus den verschiedensten Kernen bestehen, zum Beispiel organischen Polymeren oder anorganischen Materialien. Dabei bieten anorganische Nanopartikel wie Silica-Partikel den Vorteil, dass sie chemisch ausgesprochen inert und mechanisch stabil sind. Während Surfmere und molekular geprägte Polymere weiche Kerne besitzen, zeigen Nanopartikel mit Silica- oder Eisenkernen in Lösungsmitteln keine Quellung. Nicht-quellbare Partikel verändern ihre Morphologie nicht, auch wenn sie mehrmals über längere Zeit in Lösungsmitteln suspendiert werden. Erfindungsgemäße Funktionselemente, die nicht-quellbare Partikel umfassen, können daher problemlos in Analyse- oder Mikrostrukturierungsverfahren eingesetzt werden, die die Verwendung von Lösungsmitteln erfordern, ohne dass der Zustand der Nanopartikel oder der immobilisierten biologischen Moleküle nachteilig beeinflusst wird. Funktionselemente, die solche Nanopartikel enthalten, lassen sich daher auch zur Aufreinigung der zu immobilisierenden biologischen Moleküle aus komplexen Substanzgemischen, die nicht-erwünschte Substanzen wie Detergenzien oder Salze enthalten, verwenden, wobei die zu immobilisierenden Moleküle über beliebig lange Waschprozesse hinweg optimal aus solchen Substanzgemischen abgetrennt werden können. Andererseits können sich superparamagnetische oder ferromagnetische Nanopartikel mit einem Eisenoxid-Kern in einem Magnetfeld entlang der Feldlinien anordnen. Diese Eigenschaft von Eisenoxid-Nanopartikeln lässt sich benutzen, um direkt Mikrostrukturen, insbesondere nanoskopische Leiterbahnen aufzubauen.

Die erfindungsgemäßen Funktionselemente lassen sich zur Immobilisierung unterschiedlichster biologischer Moleküle verwenden, wobei deren biologische Aktivität erhalten bleibt. Die zur Bildung der Mikrostrukturen verwendeten Nanopartikel weisen molekülspezifische Erkennungsstellen, insbesondere funktionelle chemische Gruppen auf, die das zu immobilisierende Molekül so binden können, dass die für die biologische Aktivität erforderlichen Molekülbereiche in einem dem nativen Molekülzustand entsprechenden Zustand vorliegen. In Abhängigkeit von den auf der Nanopartikel-Oberfläche vorhandenen funktionellen Gruppen können die Biomoleküle je nach Bedarf kovalent und/oder nicht-kovalent an den Nanopartikeln gebunden sein. Die Nanopartikel können unterschiedliche funktionelle Gruppen aufweisen, so dass sich entweder unterschiedliche Biomoleküle oder Biomoleküle mit unterschiedlichen funktionellen Gruppen mit bevorzugter Ausrichtung immobilisieren lassen. Die Biomoleküle können sowohl ungerichtet als auch gerichtet an den Nanopartikeln immobilisiert werden, wobei nahezu jede gewünschte Ausrichtung der Biomoleküle möglich ist. Durch die Immobilisierung der Biomoleküle an den Nanopartikeln wird auch eine Stabilisierung der Biomoleküle erreicht.

Die zur Bildung der Mikrostrukturen verwendeten Nanopartikel besitzen ein vergleichsweise sehr großes Oberfläche-Volumen-Verhältnis und können dementsprechend eine große Menge eines biologischen Moleküls pro Masse binden. Im Vergleich zu Systemen, bei denen biologische Moleküle direkt an einem planaren Träger gebunden sind, kann ein Funktionselement somit pro Flächeneinheit eine erheblich größere Menge der biologischen Moleküle binden. Die Menge der pro Flächeneinheit gebundenen Molekülmenge, das heißt die Packungsdichte, kann noch dadurch gesteigert werden, dass zur Erzeugung der Mikrostruktur auf der Trägeroberfläche mehrere Partikel-Lagen übereinander geschichtet werden. Eine weitere Erhöhung der pro Flächeneinheit gebundenen Menge biologischer Moleküle kann dadurch erreicht werden, dass die Nanopartikel zunächst mit Hydrogelen und dann mit biologischen Molekülen beschichtet werden.

Die erfindungsgemäß verwendeten Nanopartikel besitzen einen Durchmesser von 5 nm bis 500 nm. Unter Verwendung solcher Nanopartikel lassen sich daher Funktionselemente herstellen, die sehr kleine Mikrostrukturen beliebiger Form im Nanometer- bis Mikrometerbereich aufweisen. Die Verwendung der Nanopartikel zur Erzeugung der Mikrostrukturen erlaubt daher eine bislang nicht erreichte Miniaturisierung der Funktionselemente, die mit erheblichen Verbesserungen signifikanter Parameter der Funktionselemente einhergeht.

Die erfindungsgemäß verwendeten Nanopartikel zeigen auf den Materialien, die zur Herstellung der Träger beziehungsweise Trägeroberflächen verwendet werden, eine sehr guten Haftung. Die Partikel können dadurch problemlos für eine Vielzahl von Trägersystemen und somit für eine Vielzahl unterschiedlicher Funktionselemente mit unterschiedlichsten Anwendungsgebereichen verwendet werden. Die unter Verwendung der Nanopartikel gebildeten Mikrostrukturen sind sehr homogen, was zu einer ortsunabhängigen Signalintensität führt.

Die erfindungsgemäßen Funktionselemente können auf ihrer Trägeroberfläche unterschiedliche Mikrostrukturen aufweisen, die aus unterschiedlichen Nanopartikeln mit unterschiedlichen molekülspezifischen Erkennungsstellen bestehen. Dementsprechend können diese unterschiedlichen Mikrostrukturen auch mit unterschiedlichen Biofunktionen belegt werden. Die Funktionselemente können also nebeneinander Mikrostrukturen enthalten, die unterschiedliche biologischen Molekülen enthalten beziehungsweise damit versehen werden können. Ein Funktionselement kann daher beispielsweise mehrere unterschiedliche Proteine oder mehrere unterschiedliche Nucleinsäuren oder gleichzeitig Proteine und Nucleinsäuren enthalten.

Die erfindungsgemäßen Funktionselemente lassen sich auf einfache Weise unter Verwendung bekannter Verfahren herstellen. Beispielsweise lassen sich unter Verwendung geeigneter Suspendiermittel aus Nanopartikeln sehr einfach stabile Suspensionen erzeugen. Nanopartikel-Suspensionen verhalten sich wie Lösungen und sind dadurch kompatibel mit Mikrostrukturierungsverfahren. Nanopartikelsuspensionen können daher direkt, beispielsweise unter Verwendung herkömmlicher Verfahren wie Nadel-Ring-Printer, lithographischer Verfahren Tintenstrahlverfahren und/oder Mikrokontaktverfahren, strukturiert auf geeignete Träger abgeschieden werden, die zuvor mit einem Verbindungsmittel zur festen Anhaftung der Nanopartikel vorbehandelt wurden. Durch eine geeignete Wahl des Verbindungsmittels kann die gebildete Mikrostruktur so ausgebildet werden, dass sie zu einem späteren Zeitpunkt teilweise oder vollständig von der Trägeroberfläche des Funktionselementes, beispielsweise durch Änderung des pH-Wertes oder der Temperatur, gelöst und gegebenenfalls auf die Trägeroberfläche eines andern Funktionselementes übertragen werden kann.

Die erfindungsgemäßen Funktionselemente können in unterschiedlichster Form ausgeführt sein und lassen sich daher auch in sehr unterschiedlichen Bereichen einsetzen. Beispielsweise kann es sich bei den erfindungsgemäßen Funktionselementen um Biochips, beispielsweise Gen- oder Protein-Arrays, handeln, die in der medizinischen Analyse oder Diagnostik eingesetzt werden. Die erfindungsgemäßen Funktionselemente lassen sich aber auch als Elektronikbaustein, beispielsweise als molekularer Schaltkreis, in der medizinischen Mess- und Überwachungstechnik oder in einem Biocomputer verwenden.

Im Zusammenhang mit der vorliegenden Erfindung wird unter einem "Funktionselement" ein Element verstanden, das entweder allein oder als Bestandteil einer komplexeren Vorrichtung, das heißt im Zusammenhang mit weiteren ähnlichen oder anders gearteten Funktionselementen, mindestens eine definierte Funktion ausübt. Ein Funktionselement umfasst mehrere Bestandteile, die aus gleichen oder unterschiedlichen Materialien bestehen können. Die einzelnen Bestandteile eines Funktionselementes können innerhalb eines Funktionselementes unterschiedliche Funktionen ausüben und können in unterschiedlichem Maße oder in unterschiedlicher Art und Weise zur Gesamtfunktion des Elementes beitragen. In der vorliegenden Erfindung umfasst ein Funktionselement einen Träger mit einer Trägeroberfläche, auf der (ein) definierte Schicht(en) von Nanopartikeln als Mikrostruktur(en) angeordnet ist/sind, wobei die Nanopartikel mit biologischen Funktionen, beispielsweise biologischen Molekülen wie Nucleinsäuren, Proteinen und/oder PNA-Molekülen versehen sind und/oder versehen werden können.

Unter einem "Träger" wird derjenige Bestandteil des Funktionselementes verstanden, der hauptsächlich das Volumen und die äußere Gestalt des Funktionselementes bestimmt. Der Begriff "Träger" bedeutet insbesondere eine feste Matrix. Der Träger kann eine beliebige Größe und eine beliebige Form aufweisen, beispielsweise die einer Kugel, eines Zylinders, einer Stange, eines Drahtes, einer Platte oder einer Folie. Der Träger kann sowohl ein Hohlkörper als auch ein Vollkörper sein. Mit einem Vollkörper ist insbesondere ein Körper gemeint, der im Wesentlichen keine Hohlräume aufweist und vollständig aus einem Material oder einer MaterialKombination bestehen kann. Der Vollkörper kann auch aus einer Schichtabfolge gleicher oder unterschiedlicher Materialien bestehen.

Erfindungsgemäß besteht der Träger des Funktionselementes, insbesondere die Träger-Oberfläche, aus einem Metall, einem Metalloxid, einem Polymer, Glas, einem Halbleitermaterial oder Keramik. Im Zusammenhang mit der Erfindung bedeutet dies, dass entweder der Träger vollständig aus einem der vorstehend genannten Materialien besteht oder dieses im Wesentlichen enthält oder vollständig aus einer Kombination dieser Materialien besteht oder diese im Wesentlichen enthält oder dass die Oberfläche des Trägers vollständig aus einem der vorstehend genannten Materialien besteht oder diese im Wesentlichen enthält oder vollständig aus einer Kombination dieser Materialien besteht oder diese im Wesentlichen enthält. Der Träger oder seine Oberfläche besteht dabei zumindest zu etwa 60%, vorzugsweise zu etwa 70%, bevorzugter zu etwa 80% und am bevorzugtesten zu etwa 100% aus einem der vorstehend genannten Materialien oder einer Kombination solcher Materialien.

In bevorzugter Ausführungsform besteht der Träger des Funktionselementes aus Materialien, wie transparentem Glas, Siliciumdioxid, Metallen, Metalloxiden, Polymeren und Copolymeren von Dextranen oder Amiden, beispielsweise Acrylamid-Derivaten, Cellulose, Nylon, oder polymeren Materialien, wie Polyethylenterephthalat, Celluloseacetat, Polystyrol oder Polymethylmethacrylat oder einem Polycarbonat von Bisphenol A.

Erfindungsgemäß ist vorgesehen, dass die Oberfläche des Funktionselement-Trägers planar oder auch vorstrukturiert ist, beispielsweise Zu- und Ableitungen enthält. Erfindungsgemäß ist ebenfalls vorgesehen, dass die von der Mikrostruktur nicht abgedeckten Flächenabschnitte der Oberfläche des Trägers Funktionalitäten beziehungsweise chemische Verbindungen enthalten, die eine unspezifische Anlagerung von Biomolekülen an diese Flächenabschnitte verhindern. Dabei handelt es sich insbesondere um eine Ethylenoxid-Schicht.

Es ist vorgesehen, dass zwischen der Trägeroberfläche und der Mikrostruktur mindestens eine Schicht eines Verbindungsmittels angeordnet ist. Das Verbindungsmittel dient zur festen Bindung der Nanopartikel an der Trägeroberfläche des Funktionselementes. Die Auswahl des Verbindungsmittels richtet sich nach der Oberfläche des Trägermaterials und den zu bindenden Nanopartikeln. Bei dem Verbindungsmittel handelt es sich vorzugsweise um geladene oder ungeladene Polymere. Bei dem Polymer kann es sich auch um ein Hydrogel handeln. Bei dem Verbindungsmittel kann es sich auch um eine Plasmaschicht mit geladenen Gruppen, wie einen Polyelektrolyten, oder eine Plasmaschicht mit chemisch reaktiven Gruppen handeln. Das Verbindungsmittel kann auch einen Self-Assembled-Monolayer auf Silan- oder Thiol-Basis sein. Erfindungsgemäß ist vorgesehen, dass die Verbindungsmittelschicht aus mindestens einer Schicht eines Verbindungsmittels besteht. Die Verbindungsmittelschicht kann jedoch auch aus mehreren Schichten unterschiedlicher Verbindungsmittel bestehen, beispielsweise aus einer anionischen Plasma-Schicht und einer kationischen Polymerschicht oder aus mehreren Polymerschichten, die abwechselnd anionisch und kationisch sind.

Eine weitere bevorzugte Ausführungsform der Erfindung betrifft Verbindungsmittel, deren Eigenschaften, beispielsweise ihre Kohäsionseigenschaften, durch einen äußeren Stimulus verändert werden können und die daher von außen schaltbar sind. Beispielsweise können die Kohäsionseigenschaften des Verbindungsmittel durch Änderung des pH-Wertes, der Ionenkonzentration und/oder der Temperatur soweit vermindert werden, dass die unter Verwendung des Verbindungsmittels an der Trägeroberfläche des Funktionselementes gebundenen Mikrostrukturen abgelöst und gegebenenfalls auf die Trägeroberfläche eines anderen Funktionselementes übertragen werden können.

In einer weiteren bevorzugten Ausführungsform der Erfindung ist vorgesehen, dass der Träger, insbesondere die Trägeroberfläche, vor dem Aufbringen der Verbindungsschichten und Mikrostrukturen mit einem Oberflächen-aktivierenden Mittel vorbehandelt wird, um die Bindung der aufzutragenden Verbindungsschichten und Mikrostrukturen am Träger beziehungsweise an dessen Oberfläche zu verbessern. Die Oberflächen des Trägers können beispielsweise mittels chemischer Verfahren, beispielsweise unter Verwendung von Primern oder einer Säure beziehungsweise einer Base, aktiviert werden. Die Oberflächenaktivierung kann auch unter Verwendung eines Plasmas erfolgen. Die Oberflächenaktivierung kann auch das Aufbringen eines Self-Assembled-Monolayers umfassen.

Unter einer "Mikrostruktur" werden Strukturen im Bereich einzelner Mikrometer oder Nanometer verstanden. Insbesondere im Zusammenhang mit der vorliegenden Erfindung wird unter "Mikrostruktur" eine Struktur verstanden, die aus mindestens zwei Einzelkomponenten in Form von Nanopartikeln mit molekülspezifischen Erkennungsstellen besteht und auf der Oberfläche eines Trägers angeordnet ist, wobei ein bestimmter Flächenabschnitt der Oberfläche des Trägers abgedeckt wird, der eine definierte Form und einen definierten Flächeninhalt aufweist und der kleiner als die Träger-Oberfläche ist. Erfindungsgemäß ist insbesondere vorgesehen, dass mindestens einer der Flächen-Längen-Parameter, der den durch die Mikrostruktur abgedeckten Flächenabschnitt bestimmt, im Mikrometerbereich liegt. Weist die Mikrostruktur beispielsweise die Form eines Kreises auf, liegt der Durchmesser des Kreises im Mikrometerbereich. Ist die Mikrostruktur als Rechteck ausgeführt, liegt beispielsweise die Breite dieses Rechteckes im Mikrometerbereich. Erfindungsgemäß ist insbesondere vorgesehen, dass der mindestens eine Flächen-Längen-Parameter, der den von der Mikrostruktur abgedeckten Flächenabschnitt bestimmt, kleiner als 1 mm ist. Da die Mikrostruktur erfindungsgemäß aus mindestens zwei Nanopartikeln besteht, liegt die untere Grenze dieses Flächen-Längenparameters bei 10 nm.

Der von der erfindungsgemäßen Mikrostruktur abgedeckte Flächenabschnitt kann eine beliebige geometrische Form aufweisen, beispielsweise die eines Kreises, einer Ellipse, eines Quadrates, eines Rechteckes oder einer Linie. Die Mikrostruktur kann aber auch aus mehreren regelmäßigen und/oder unregelmäßigen geometrischen Formen zusammengesetzt sein. Handelt es sich bei dem erfindungsgemäßen Funktionselement beispielsweise um einen Gen-Chip oder ein Protein-Array, so weist die Mikrostruktur vorzugsweise eine kreis- oder ellipsenähnliche Form auf. Handelt es sich bei dem erfindungsgemäßen Funktionselement um einen Elektronikbaustein zur Verwendung in einem Biocomputer, kann die Mikrostruktur auch eine Schaltkreis-ähnliche Form aufweisen. Erfindungsgemäß ist auch vorgesehen, dass auf der Trägeroberfläche eines Funktionselementes mehrere Mikrostrukturen gleicher oder unterschiedlicher Form mit einem Abstand zueinander angeordnet sind.

Erfindungsgemäß ist vorgesehen, dass die Mikrostrukturen beispielsweise unter Verwendung eines Nadel-Ring-Printers per Ring/Pin mittels lithografischer Verfahren, wie Photolithographie oder Mikropen-Lithographie, Tintenstrahltechniken oder Mikrokontaktdruckverfahren auf die Oberfläche des Funktionselement-Trägers aufgetragen werden. Die Auswahl des Verfahrens, mit dessen Hilfe die Mikrostruktur beziehungsweise Mikrostrukturen auf die Oberfläche des Funktionselementes aufgebracht werden, richtet sich nach der Oberfläche des Trägermaterials, den Nanopartikeln, die die Mikrostruktur bilden sollen, und der späteren Anwendung des Funktionselementes.

Im Zusammenhang mit der vorliegenden Erfindung wird unter einem "Nanopartikel" eine partikuläre Bindematrix verstanden, die erste funktionelle chemische Gruppen umfassende molekülspezifische Erkennungsstellen aufweist. Die erfindungsgemäß verwendeten Nanopartikel umfassen einen Kern mit einer Oberfläche, auf der die ersten funktionellen Gruppen angeordnet sind, die in der Lage sind, komplementäre zweite funktionelle Gruppen eines Biomoleküls kovalent oder nicht-kovalent zu binden. Durch Wechselwirkung zwischen den ersten und zweiten funktionellen Gruppen ist das Biomolekül an dem Nanopartikel und damit an der Mikrostruktur des Funktionselementes immobilisiert und/oder kann daran immobilisiert werden. Die erfindungsgemäß zur Bildung der Mikrostrukturen verwendeten Nanopartikel weisen eine Größe von kleiner 500 nm, vorzugsweise kleiner 150 nm auf.

Im Zusammenhang mit der vorliegenden Erfindung bedeutet "adressierbar", dass die Mikrostruktur nach dem Auftragen der Nanopartikel auf die Trägeroberfläche wieder auffindbar und/oder nachweisbar ist. Wird die Mikrostruktur beispielsweise unter Verwendung einer Maske oder eines Stempels auf die Trägeroberfläche aufgetragen, resultiert die Adresse der Mikrostruktur einerseits aus den Koordinaten x und y des von der Maske oder dem Stempel vorgegebenen Bereiches der Trägeroberfläche, auf den die Mikrostruktur aufgetragen wird. Andererseits resultiert die Adresse der Mikrostruktur aus den molekülspezifischen Erkennungsstellen auf der Oberfläche der Nanopartikel, die ein Wiederauffinden beziehungsweise einen Nachweis der Mikrostruktur erlauben. Wenn die Mikrostruktur biofunktionalisierbar ist, das heißt Nanopartikel mit molekülspezifischen Erkennungsstellen umfasst, an die keine Biomoleküle gebunden sind, kann die Mikrostruktur dadurch wieder aufgefunden und/oder nachgewiesen werden, dass ein oder mehrere Biomoleküle spezifisch an die molekülspezifischen Erkennungsstellen der die Mikrostruktur bildenden Nanopartikel binden, nicht jedoch an die Flächenabschnitte der Trägeroberfläche, die nicht von der Mikrostruktur abgedeckt sind. Wenn das immobilisierte Molekül beispielsweise mit Detektionsmarkern wie Fluorophoren, Spin-Labeln, Goldpartikeln, radioaktiven Markern etc., markiert ist, kann der Nachweis der Mikrostruktur unter Verwendung entsprechend geeigneter Nachweisverfahren erfolgen. Wenn die Mikrostruktur biofunktionalisiert ist, das heißt Nanopartikel umfasst, an deren molekülspezifische Erkennungsstellen bereits ein oder mehrere Biomoleküle gebunden sind, bedeutet "addressierbar", dass diese Biomoleküle durch Wechselwirkung mit komplementären Strukturen weiterer Moleküle oder mittels messtechnischer Verfahren aufgefunden und/oder nachgewiesen werden können, wobei nur die aus Nanopartikeln bestehende Mikrostruktur entsprechende Signale zeigt, nicht jedoch die Flächenabschnitte der Trägeroberfläche, die nicht von der Mikrostruktur abgedeckt sind. Als Nachweisverfahren kann beispielsweise die Matrix-unterstützte Laser-Desorptions/Ionisations-Flugzeit-Massenspektrometrie (MALDI-TOF-MS) eingesetzt werden, die sich zu einem wichtigen Verfahren zur Analyse von unterschiedlichsten Substanzen, insbesondere Proteinen, entwickelt hat. Weitere Nachweisverfahren sind Wellenleiterspektroskopie, Fluoreszenz, Impedanzspektroskopie, radiometrische und elektrische Verfahren.

Erfindungsgemäß ist vorgesehen, dass das biologische Molekül unter Erhalt seiner biologischen Aktivität an der Oberfläche der die Mikrostruktur bildenden Nanopartikel gebunden oder immobilisiert ist oder gebunden oder immobilisiert werden kann, wobei das Molekül vorzugsweise gerichtet gebunden ist oder wird. Unter der biologischen Aktivität eines Moleküls werden alle Funktionen verstanden, die dieses in einem Organismus in seiner natürlichen zellulären Umgebung ausübt. Wenn das Molekül ein Protein ist, kann es sich um spezifische katalytische oder enzymatische Funktionen, Funktionen in der Immunabwehr, Transport- und Speicherfunktion, Regulationsfunktion, Transkriptions- und Translationsfunktionen und ähnliche handeln. Handelt es sich bei dem Molekül um eine Nucleinsäure, kann die biologische Funktion beispielsweise in der Codierung eines Genproduktes bestehen oder darin, dass die Nucleinsäure als Matrize zur Synthese weiterer Nucleinsäuremoleküle oder als Bindungsmotiv für regulatorische Proteine verwendbar ist. "Beibehaltung der biologischen Aktivität" bedeutet, dass ein biologisches Molekül nach Immobilisierung an der Oberfläche eines Nanopartikels die gleichen oder nahezu gleichen biologischen Funktionen in zumindest ähnlichem Ausmaß ausüben kann wie das gleiche Molekül in nicht-immobilisiertem Zustand unter geeigneten in vitro-Bedingungen beziehungsweise das gleiche Molekül in seiner natürlichen zellulären Umgebung. Im Zusammenhang mit der vorliegenden Erfindung bedeutet der Begriff "gerichtet immobilisiert" oder "gerichtete Immobilisierung", dass ein Molekül an definierten Positionen innerhalb des Moleküls so an den molekülspezifischen Erkennungssequenzen eines Nanopartikel gebunden wird beziehungsweise ist, dass beispielsweise die dreidimensionale Struktur der für die biologische Aktivität erforderlichen Domäne(n) gegenüber dem nicht-immobilisierten Zustand nicht verändert ist und dass diese Domäne(n), beispielsweise Bindungstaschen für zelluläre Reaktionspartner, bei Kontakt mit anderen nativen zellulären Reaktionspartnern für diese frei zugänglich ist/sind.

"Gerichtet immobilisiert" bedeutet auch, dass bei der Immobilisierung einer Molekülspezies alle oder nahezu alle individuellen Moleküle, mindestens jedoch mehr als 80%, vorzugsweise mehr als 85% aller Moleküle auf der Oberfläche der die Mikrostruktur bildenden Nanopartikel eine identische oder nahezu identische Ausrichtung reproduzierbar einnehmen.

Erfindungsgemäß ist vorgesehen, dass das an der Mikrostruktur des erfindungsgemäßen Funktionselementes immobilisierte oder immobilisierbare biologische Molekül insbesondere eine Nucleinsäure, ein Protein, ein PNA-Molekül, ein Fragment davon oder ein Gemisch davon ist.

Im Zusammenhang mit der vorliegenden Erfindung wird unter einer Nucleinsäure ein Molekül verstanden, das aus mindestens zwei über eine Phosphodiesterbindung verbundenen Nucleotiden besteht. Bei Nucleinsäuren kann es sich sowohl um eine Desoxyribonucleinsäure als auch eine Ribonucleinsäure handeln. Die Nucleinsäure kann sowohl einsträngig als auch zweisträngig vorliegen. Im Kontext der vorliegenden Erfindung kann eine Nucleinsäure also auch ein Oligonucleotid sein. Die an der Mikrostruktur des erfindungsgemäßen Funktionselementes gebundene Nucleinsäure weist vorzugsweise eine Länge von mindestens 10 Basen auf. Eine Nucleinsäure kann natürlichen oder synthetischen Ursprungs sein. Die Nucleinsäure kann durch gentechnische Verfahren gegenüber der Wildtyp-Nucleinsäure modifiziert sein und/oder unnatürliche und/oder ungewöhnliche Nucleinsäurebausteine enthalten. Die Nucleinsäure kann mit Molekülen anderer Art, beispielsweise mit Proteinen, verbunden sein.

Im Zusammenhang mit der vorliegenden Erfindung wird unter einem "Protein" ein Molekül verstanden, das mindestens zwei über eine Amidbindung miteinander verbundene Aminosäuren umfasst. Im Kontext der vorliegenden Erfindung kann ein Protein also auch ein Peptid, zum Beispiel ein Oligopeptid, ein Polypeptid oder zum Beispiel eine Protein-Domäne sein. Ein derartiges Protein kann natürlichen oder synthetischen Ursprungs sein. Das Protein kann durch gentechnische Verfahren gegenüber dem Wildtyp-Protein modifiziert sein und/oder unnatürliche und/oder ungewöhnliche Aminosäuren enthalten. Das Protein kann gegenüber der Wildtyp-Form derivatisiert sein, beispielsweise Glykosylierungen aufweisen, es kann verkürzt sein, es kann mit anderen Proteinen fusioniert sein oder mit Molekülen anderer Art, beispielsweise mit Kohlenhydraten, verbunden sein. Erfindungsgemäß kann ein Protein insbesondere ein Enzym, ein Rezeptor, ein Cytokin, ein Antigen oder ein Antikörper sein.

"Antikörper" bedeutet ein Polypeptid, das im wesentlichen von einem oder mehreren Immunglobulin-Genen codiert wird, oder Fragmente davon, das/die einen Analyten (Antigen) spezifisch bindet/binden und erkennt/erkennen. Antikörper kommen beispielsweise als intakte Immunglobuline oder als eine Reihe von Fragmenten vor, die mittels Spaltung mit verschiedenen Peptidasen erzeugt werden. "Antikörper" bedeutet auch modifizierte Antikörper (z.B. oligomere, reduzierte, oxidierte und markierte Antikörper). "Antikörper" umfasst auch Antikörper-Fragmente, die entweder mittels Modifikation ganzer Antikörper oder mittels de novo-Synthese unter Verwendung von DNA-Rekombinationstechniken erzeugt worden sind. Der Begriff "Antikörper" umfaßt sowohl intakte Moleküle als auch Fragmente davon, wie Fab, F(ab')₂ und Fv, die die Epitop-Determinante binden können.

Bei PNA (Peptide Nucleic Acid oder Polyamide Nucleic Acid)-Molekülen handelt es sich um Moleküle, die nicht negativ geladen sind und in gleicher Weise wie DNA wirken (Nielsen et al., 1991, Science, 254, 1497-1500; Nielsen et al., 1997, Biochemistry, 36, 5072-5077; Weiler et al., 1997, Nuc. Acids Res., 25, 2792-2799). PNA-Sequenzen umfassen ein Polyamid-Grundgerüst aus N-(2-Aminoethyl)-glycin-Einheiten und besitzen keine Glucose-Einheiten und keine Phosphat-Gruppen.

Im Zusammenhang mit der vorliegenden Erfindung werden unter "molekülspezifischen Erkennungsstellen" Bereiche des Nanopartikels verstanden, die eine spezifische Wechselwirkung zwischen dem Nanopartikel und biologischen Molekülen als Zielmolekülen ermöglichen. Die Wechselwirkung kann auf gerichteter attraktiver Wechselwirkung zwischen einem oder mehreren Paaren aus ersten funktionellen Gruppen des Nanopartikels und die ersten funktionellen Gruppen bindenden, komplementären zweiten funktionellen Gruppen der Zielmoleküle, also der biologischen Moleküle, beruhen. Einzelne interagierende Paare funktioneller Gruppen zwischen Nanopartikel und biologischem Molekül sind jeweils räumlich fixiert an dem Nanopartikel und dem biologischen Molekül angeordnet. Diese Fixierung braucht keine starre Anordnung zu sein, sondern kann vielmehr durchaus flexibel ausgeführt sein. Die attraktive Wechselwirkung zwischen den funktionellen Gruppen der Nanopartikel und der biologischen Moleküle kann in Form von nicht-kovalenten Bindungen wie van-der-Waals-Bindungen, Wasserstoffbrücken-Bindungen, π-π-Bindungen, elektrostatischen Wechselwirkungen oder hydrophoben Wechselwirkungen ausgeführt sein. Denkbar sind auch reversible kovalente Bindungen ebenso wie Mechanismen, die auf Komplementarität der Gestalt oder Form beruhen. Die erfindungsgemäß vorgesehenen Wechselwirkungen zwischen den molekülspezifischen Erkennungsstellen der Nanopartikel und dem Zielmolekül beruhen also auf gerichteten Wechselwirkungen zwischen den Paaren der funktionellen Gruppen und auf der räumlichen Anordnung dieser die Paarbildung eingehenden Gruppen zueinander an dem Nanopartikel sowie dem Zielmolekül. Diese Wechselwirkung führt zu einer affinen Bindung kovalenter oder nicht-kovalenter Art zwischen den beiden Bindungspartnern, dergestalt, dass das biologische Molekül an der Oberfläche der die Mikrostruktur bildenden Nanopartikel immobilisiert wird.

In einer bevorzugten Ausführungsform der vorliegenden Erfindung sind die ersten funktionellen Gruppen, die Bestandteil der molekülspezifischen Erkennungsstellen auf der Oberfläche des Nanopartikels sind oder diese bilden, ausgewählt aus der Gruppe bestehend aus Aktivester, Alkylketongruppe, Aldehydgruppe, Aminogruppe, Carboxygruppe, Epoxygruppe, Maleinimidogruppe, Hydrazingruppe, Hydrazidgruppe, Thiolgruppe, Thioestergruppe, Oligohistidingruppe, Strep-Tag I, Strep-Tag II, Desthiobiotin, Biotin, Chitin, Chitinderivate, Chitinbindedomäne, Metallchelatkomplex, Streptavidin, Streptactin, Avidin und Neutravidin.

Erfindungsgemäß ist auch vorgesehen, dass die molekülspezifische Erkennungsstelle ein größeres Molekül wie ein Protein, ein Antikörper etc. ist, das die ersten funktionellen Gruppen enthält. Die molekülspezifische Erkennungsstelle kann auch ein Molekülkomplex sein, der aus mehreren Proteinen und/oder Antikörpern und/oder Nucleinsäuren besteht, wobei mindestens eines dieser Moleküle die ersten funktionellen Gruppen enthält. Ein Protein kann als molekülspezifische Erkennungssequenz beispielsweise einen Antikörper und ein damit verbundenes Protein umfassen. Der Antikörper kann dabei auch eine Streptavidin-Gruppe oder eine Biotin-Gruppe umfassen. Bei dem mit dem Antikörper verbundenen Protein kann es sich um einen Rezeptor handeln, beispielsweise ein MHC-Protein, Cytokin, einen T-Zell-Rezeptoren wie das CD-8-Protein und andere, der einen Liganden binden kann. Ein Molekülkomplex kann auch mehrere Proteine und/oder Peptide umfassen, beispielsweise ein biotinyliertes Protein, das in einem Komplex ein weiteres Protein und zusätzlich ein Peptid bindet.

Die zweite funktionelle Gruppe, also die funktionelle Gruppe des zu immobilisierenden Biomoleküls, ist erfindungsgemäß ausgewählt aus der Gruppe bestehend aus aus der Gruppe bestehend aus Aktivester, Alkylketongruppe, Aldehydgruppe, Aminogruppe, Carboxygruppe, Epoxygruppe, Maleinimidogruppe, Hydrazingruppe, Hydrazidgruppe, Thiolgruppe, Thioestergruppe, . Oligohistidingruppe, Strep-Tag I, Strep-Tag II, Desthiobiotin, Biotin, Chitin, Chitinderivate, Chitinbindedomäne, Metallchelatkomplex, Streptavidin, Streptactin, Avidin und Neutravidin.

Die ersten und zweiten funktionellen Gruppen können beispielsweise durch molekulares Prägen erzeugt worden sein. Die ersten und zweiten funktionellen Gruppen können auch Aktivester, wie die sogenannten Surfmere sein.

Ein erfindungsgemäß verwendetes Nanopartikel weist also an seiner Oberfläche eine erste funktionelle Gruppe auf, die kovalent oder nicht-kovalent mit einer zweiten funktionellen Gruppe eines zu immobilisierenden Biomoleküls verknüpft wird, wobei die erste funktionelle Gruppe eine andere Gruppe als die zweite funktionelle Gruppe ist. Die beiden miteinander in Bindung tretenden Gruppen müssen komplementär zueinander sein, das heißt in der Lage sein, eine kovalente oder nicht-kovalente Bindung miteinander einzugehen.

Wird erfindungsgemäß als erste funktionelle Gruppe beispielsweise eine Alkylketongruppe, insbesondere Methylketon- oder Aldehydgruppe verwendet, ist die zweite funktionelle Gruppe eine Hydrazin- oder Hydrazidgruppe. Wird umgekehrt eine Hydrazin- oder Hydrazidgruppe als erste funktionelle Gruppe verwendet, ist erfindungsgemäß die zweite funktionelle Gruppe eine Alkylketon, insbesondere Methylketon- oder Aldehydgruppe. Wird erfindungsgemäß eine Thiolgruppe als erste funktionelle Gruppe verwendet, ist die zweite komplementäre funktionelle Gruppe eine Thioestergruppe. Wird als erste funktionelle Gruppe eine Thioestergruppe verwendet, ist erfindungsgemäß die zweite funktionelle Gruppe eine Thiolgruppe.

Wird erfindungsgemäß als erste funktionelle Gruppe ein Metallionenchelatkomplex verwendet, ist die zweite funktionelle komplementäre Gruppe eine Oligohistidingruppe. Wird als erste funktionelle Gruppe eine Oligohistidingruppe verwendet, ist die zweite funktionelle komplementäre Gruppe ein Metalionenchelatkomplex.

Wird als erste funktionelle Gruppe Strep-Tag I, Strep-Tag II, Biotin oder Desthiobiotin verwendet, wird als zweite komplementäre funktionelle Gruppe Streptavidin, Streptactin, Avidin oder Neutravidin eingesetzt. Wird als erste funktionelle Gruppe Streptavidin, Streptactin, Avidin oder Neutravidin eingesetzt, wird als zweite komplementäre funktionelle Gruppe Strep-Tag I, Strep-Tag II, Biotin oder Desthiobiotin eingesetzt.

Wird in einer weiteren Ausführungsform Chitin oder ein Chitinderivat als erste funktionelle Gruppe eingesetzt, wird als zweite funktionelle komplementäre Gruppe eine Chitinbindungsdomäne eingesetzt. Wird als erste funktionelle Gruppe eine Chitinbindungsdomäne eingesetzt, wird als zweite funktionelle komplementäre Gruppe Chitin oder ein Chitinderivat eingesetzt.

Die vorgenannten ersten und/oder zweiten funktionellen Gruppen können erfindungsgemäß mit Hilfe eines Spacers mit dem zu immobilisierenden Biomolekül beziehungsweise dem Nanopartikel-Kern verbunden beziehungsweise mittels eines Spacers an den Nanopartikel-Kern oder in das Biomolekül eingeführt werden. Der Spacer dient also einerseits als Abstandshalter der funktionellen Gruppe zum Kern beziehungsweise Biomolekül, andererseits als Träger für die funktionelle Gruppe. Ein derartiger Spacer kann erfindungsgemäß Alkylen-Gruppen oder Ethylenoxid-Oligomere mit 2 bis 50 C-Atomen darstellen, der in bevorzugter Ausführungsform substituiert ist und Heteroatome aufweist.

In bevorzugter Ausführungsform der Erfindung ist vorgesehen, dass die zweiten funktionellen Gruppen ein natürlicher Bestandteil des Biomoleküls, insbesondere eines Proteins, sind.

Bei einem Protein mittlerer Größe, also einer Größe von etwa 50 kDA mit etwa 500 Aminosäuren, gibt es etwa 20 bis 30 reaktive Amino-Gruppen, die prinzipiell als funktionelle Gruppe zur Immobilisierung in Frage kommen. Insbesondere handelt es sich dabei um die Amino-Gruppe am N-terminalen Ende eines Proteins. Auch alle anderen freien Amino-Gruppen, insbesondere die der Lysinreste, kommen für die Immobilisierung in Frage. Auch Arginin mit seiner Guanidium-Gruppe kommt als funktionelle Gruppe in Betracht.

In einer weiteren bevorzugten Ausführungsform der Erfindung ist vorgesehen, die zweiten funktionellen Gruppen mittels gentechnischer Verfahren, biochemischer, enzymatischer und/oder chemischer Derivatisierung oder chemischer Syntheseverfahren in das Biomolekül einzuführen. Die Derivatisierung sollte so erfolgen, dass die biologische Aktivität nach der Immobilisierung erhalten bleibt.

Ist das Biomolekül ein Protein, können beispielsweise unnatürliche Aminosäuren durch gentechnische Verfahren oder während einer chemischen Proteinsynthese in das Proteinmolekül eingefügt werden, beispielsweise zusammen mit Spacern oder Linkern. Derartige unnatürliche Aminosäuren sind Verbindungen, die eine Aminosäurefunktion und einen Rest R aufweisen und nicht über einen natürlich vorkommenden genetischen Code definiert sind, wobei diese Aminosäuren in besonders bevorzugter Weise eine ThiolGruppe aufweisen. Erfindungsgemäß kann auch vorgesehen sein, eine natürlicherweise vorkommende Aminosäure, beispielsweise Lysin, zu modifizieren, zum Beispiel durch Derivatisierung ihrer Seitenkette, insbesondere deren primärer Aminogruppe, mit der Carbonsäurefunktion von Lävulinsäure.

In einer weiteren bevorzugten Ausführungsform der vorliegenden Erfindung können funktionelle Gruppen durch Modifikation eines Proteins in dieses eingeführt werden, wobei dem Protein Tags, also Markierungen, hinzugefügt werden, vorzugsweise an den C-Terminus oder den N-Terminus. Diese Tags können jedoch auch intramolekular angeordnet sein. Insbesondere ist vorgesehen, dass ein Protein dadurch modifiziert wird, dass mindestens ein Strep-Tag, beispielsweise ein Strep-Tag I oder Strep-Tag II oder Biotin hinzugefügt wird. Erfindungsgemäß werden unter einem Strep-Tag auch funktionelle und/oder strukturelle Äquivalente verstanden, sofern sie Streptavidin-Gruppen und/oder dessen Äquivalente binden können. Der Begriff "Streptavidin" erfasst im Sinne der vorliegenden Erfindung also auch dessen funktionelle und/oder strukturelle Äquivalente. Erfindungsgemäß ist auch vorgesehen, ein Protein durch Hinzufügen von einem His-Tag, das mindestens drei Histidin-Reste, vorzugsweise jedoch eine Oligohistidin-Gruppe umfasst, zu modifizieren. Der in das Protein eingeführte His-Tag kann dann an eine einen Metallchelatkomplex umfassende molekülspezifische Erkennungsstelle binden.

In einer bevorzugten Ausführungsform der Erfindung ist also vorgesehen, Proteine, die beispielsweise mit unnatürlichen Aminosäuren, natürlichen, aber unnatürlich derivatisierten Aminosäuren oder spezifischen Strep-Tags modifiziert sind, oder Antikörper-gebundene Proteine mit dazu komplementär reaktiven Nanopartikel-Oberflächen derart zur Bindung zu bringen, dass eine geeignete spezifische, insbesondere nicht-kovalente Anbindung der Proteine und damit eine gerichtete Immobilisierung der Proteine an die Oberflächen erfolgt. Nach der Ausrichtung der bioaktiven Moleküle über Tag-Bindestellen können diese Moleküle zusätzlich kovalent gebunden werden, beispielsweise auch mit einem Crosslinker wie Glutardialdehyd. Dadurch werden die Protein-Oberflächen stabiler.

Die zur Bildung einer Mikrostruktur auf der Trägeroberfläche des Funktionselementes abgeschiedenen Nanopartikel wesen neben der Oberfläche mit den molekülspezifische Erkennungsstellen einen Kern auf. Im Zusammenhang mit der vorliegenden Erfindung wird unter einem "Kern" eines Nanopartikels ein chemisch inerter Stoff verstanden, der als Träger für das zu immobilisierende Molekül dient. Erfindungsgemäß ist der Kern ein kompaktes oder hohles Partikel mit einer Größe von 5 nm bis 500 nm.

In bevorzugter Ausführungsform der vorliegenden Erfindung besteht der Kern der erfindungsgemäß verwendeten Nanopartikel aus einem anorganischen Material wie einem Metall, beispielsweise Au, Ag oder Ni, Silicium, SiO₂, SiO, einem Silikat, Al₂O₃, SiO₂·Al₂O₃, Fe₂O₃, Ag₂O, TiO₂, ZrO₂, Zr₂O₃, Ta₂O₅, Zeolith, Glas, Indiumzinnoxid, Hydroxylapatit, einem Q-Dot oder einem Gemisch davon oder enthält dieses.

In einer weiteren bevorzugter Ausführungsform der Erfindung besteht der Kern aus einem organischen Material oder enthält dieses. Vorzugsweise ist das organische Polymer Polypropylen, Polystyrol, Polyacrylat, ein Polyester von Milchsäure oder ein Gemisch davon.

Die Herstellung der Kerne der erfindungsgemäß verwendeten Nanopartikel kann unter Verwendung üblicher, auf dem Fachgebiet bekannter Verfahren, wie Sol-Gel-Synthese-Verfahren, Emulsionspolymerisation, Suspensionspolymerisation usw. erfolgen. Nach Herstellung der Kerne werden die Oberflächen der Kerne durch chemische Modifizierungsreaktionen mit den spezifischen ersten funktionellen Gruppen versehen, beispielsweise unter Verwendung üblicher Verfahren wie Pfropf-Polymerisation, Silanisierung, chemischer Derivatisierung etc. Eine Möglichkeit, oberflächenmodifizierte Nanopartikel in einem Schritt zu erzeugen, besteht im Einsatz von Surfmeren in der Emulsionspolymerisation. Eine weitere Möglichkeit ist das molekulare Prägen.

Unter molekularem Prägen wird die Polymerisation von Monomeren in Gegenwart von Templaten verstanden, die mit dem Monomer einen während der Polymerisation relativ stabilen Komplex bilden können. Nach dem Auswaschen der Template können die so hergestellten Materialien Templatmoleküle, den Templatmolekülen strukturverwandte Molekülspezies oder Moleküle, die den Templatmolekülen oder Teilen davon strukturverwandte oder identische Gruppen aufweisen, wieder spezifisch binden. Ein Templat ist daher eine in der Monomermischung während der Polymerisation vorhandene Substanz, zu der das gebildete Polymer eine Affinität aufweist.

Erfindungsgemäß besonders bevorzugt erfolgt die Herstellung oberflächenmodifizierter Nanopartikel mittels Emulsionspolymerisation unter Einsatz von Surfmeren. Surfmere sind amphiphile Monomere (Surfmer = Surfactant + Monomer), die auf der Oberfläche von Latexpartikeln einpolymerisiert werden können und diese stabilisieren. Reaktive Surfmere verfügen zusätzlich über funktionalisierbare Endgruppen, die unter milden Bedingungen mit Nucleophilen, wie primären Aminen (Aminosäuren, Peptiden, Proteinen), Thiolen oder Alkoholen umgesetzt werden können. Auf diese Weise ist eine Vielzahl biologisch aktiver polymerer Nanopartikel zugänglich. Druckschriften, die den Stand der Technik sowie Möglichkeiten und Grenzen der Anwendung von Surfmeren wiedergeben, sind US 5,177,165, US 5,525,691, US 5,162,475, US 5,827,927 und JP 4 018 929. Arbeiten zur Synthese von Surfmeren mit reaktiven Endgruppen wurden unter anderem von Nagai et al. (Polymer 1996, 37 (1), 1257-1266; Journal of Colloid and Interface Science 1995, 172, 63-70), Asua et al. (J. Applied Polym. Sci. 1997, 66, 1803-1820) und Guyot et al. (Curr. Opin. Colloid Interface Sci. 1996, 1(5), 580-586) veröffentlicht.

Die Dichte der ersten funktionellen Gruppen und der Abstand dieser Gruppen zueinander können erfindungsgemäß für jedes zu immobilisierende Molekül optimiert werden. Auch die Umgebung der ersten funktionellen Gruppen auf der Oberfläche kann im Hinblick auf eine möglichst spezifische Immobilisierung eines Biomoleküls in entsprechender Weise vorbereitet werden.

In einer bevorzugten Ausgestaltung der Erfindung ist vorgesehen, dass im Kern zusätzliche Funktionen verankert sind, die unter Verwendung geeigneter Nachweisverfahren eine einfache Detektion der Nanopartikel-Kerne und damit der Mikrostrukturen ermöglichen. Bei diesen Funktionen kann es sich beispielsweise um Fluoreszenzmarkierungen, UV/Vis-Markierungen, superparamagnetische Funktionen, ferromagnetische Funktionen und/oder radioaktive Markierungen handeln. Geeignete Verfahren zum Nachweis von Nanopartikeln umfassen beispielsweise Fluoreszenz- oder UV-Vis-Spektroskopie-, Fluoreszenz- oder Licht-Mikroskopie-, MALDI-Massenspektroskopie-, Wellenleiterspektroskopie-, Impedanzspektroskopie-, elektrische und radiometrische Verfahren. In einer weiteren Ausführungsform ist vorgesehen, dass die Kern-Oberfläche durch Aufbringen zusätzlicher Funktionen wie Fluoreszenzmarkierungen, UV/Vis-Markierungen, superparamagnetischer Funktionen, ferromagnetischer Funktionen und/oder radioaktiver Markierungen modifiziert sein kann. In noch einer weiteren Ausführungsform der Erfindung ist vorgesehen, dass der Kern der Nanopartikel mit einer organischen oder anorganischen Schicht oberflächenmodifiziert sein, die die ersten funktionellen Gruppen und die vorstehend beschriebenen zusätzlichen Funktionen aufweist.

In einer anderen Ausführungsform der Erfindung ist vorgesehen, dass die Kern-Oberfläche chemische Verbindungen aufweist, die zur sterischen Stabilisierung und/oder zur Verhinderung einer Konformationsänderung der immobilisierten Moleküle und/oder zur Verhinderung der Anlagerung weiterer biologisch aktiver Verbindungen an die Kern-Oberfläche dient. Vorzugsweise handelt es sich bei diesen chemischen Verbindungen um Polyethylenglykole, Oligoethylenglykole, Dextran oder ein Gemisch davon.

Erfindungsgemäß besteht auch die Möglichkeit, dass auf der Oberfläche der Nanopartikel-Kerne separat oder zusätzlich Ionenaustausch-Funktionen verankert sind. Nanopartikel mit Ionenaustausch-Funktionen sind insbesondere zur Optimierung der MALDI-Analyse geeignet, da dadurch störende Ionen gebunden werden können.

In einer weiteren Ausführungsform der Erfindung ist vorgesehen, dass das an der Mikrostruktur des Funktionselementes immobilisierte biologische Molekül Markierungen aufweist, die eine einfache Detektion der an der Mikrostruktur immobilisierten biologischen Moleküle unter Verwendung geeigneter Nachweisverfahren ermöglichen. Bei diesen Markierungen kann es sich beispielsweise um eine Fluoreszenzmarkierung, eine UV/Vis-Markierung, eine superparamagnetische Funktion, eine ferromagnetische Funktion und/oder eine radioaktive Markierung handeln. Wie vorstehend ausgeführt, kommen als Nachweisverfahren für diese Markierungen beispielsweise Fluoreszenz- oder UV-VIS-Spektroskopie, MALDI-Massenspektroskopie-, Wellenleiterspektroskopie-, Impedanzspektroskopie-, elektrische und radiometrische Verfahren in Betracht.

Eine weitere Ausführungsform der Erfindung betrifft ein Funktionselement mit mindestens einem an der Mikrostruktur immobilisierten biologischen Molekül, wobei an dieses immobilisierte Molekül mindestens ein weiteres biologisches Molekül kovalent oder nicht-kovalent gebunden ist. Wenn das an der Mikrostruktur immobilisierte Molekül ein Protein ist, kann daran beispielsweise ein zweites Protein oder ein Antikörper, beispielsweise durch Protein-Protein-Interaktion oder durch Antikörper-Antigen-Bindung, gebunden sein. Wenn das an der Mikrostruktur immobilisierte Molekül eine Nucleinsäure ist, kann daran beispielsweise ein Protein gebunden sein.

Eine weitere bevorzugte Ausführungsform der Erfindung betrifft ein Funktionselement, dessen Mikrostruktur(en) aus einer einzigen Nanopartikelschicht besteht. Eine weitere bevorzugte Ausführungsform der Erfindung betrifft ein Funktionselement, dessen Mikrostruktur(en) aus mehreren übereinandergelagerten Schichten der gleichen Nanopartikel besteht, wobei jede einzelne Schicht über die bereits beschriebenen Verbindungsschichten aus geeigneten Polymeren fest an die darunter liegende Schicht gebunden wird.

Noch eine weitere bevorzugte Ausführungsform der Erfindung betrifft ein Funktionselement, auf dessen Trägeroberfläche nebeneinander mehrere unterschiedliche Mikrostrukturen angeordnet sind, die aus Nanopartikeln mit unterschiedlichen molekülspezifische Erkennungsstellen bestehen. Derartige Funktionselemente enthalten daher nebeneinander Mikrostrukturen, an die unterschiedliche biologische Moleküle immobilisiert sind oder immobilisiert werden können. Die Trägeroberfläche solcher Funktionselemente kann daher beispielsweise gleichzeitig Mikrostrukturen umfassen, an denen Proteine immobilisiert sind oder werden können, und Mikrostrukturen, an denen Nucleinsäuren immobilisiert sind oder werden können. Das Funktionselement kann aber auch Mikrostrukturen aufweisen, an die unterschiedliche Proteine oder unterschiedliche Nucleinsäuren gleichzeitig immobilisiert sind oder werden können.

Eine weitere Ausführungsform der Erfindung betrifft ein Funktionselement, bei dem die Flächenabschnitte der Trägeroberfläche, die nicht von der Mikrostruktur abgedeckt sind, durch Aufbringen zusätzlicher Funktionalitäten beziehungsweise chemischer Verbindungen modifiziert sind. Dabei kann es sich insbesondere, um Funktionalitäten beziehungsweise chemische Verbindungen handeln, die eine unspezifische Anlagerung von Biomolekülen an die nicht von der Mikrostruktur abgedeckten Bereiche der Trägeroberfläche verhindern. Vorzugsweise handelt es sich bei diesen chemischen Verbindungen um Polyethylenglykole, Oligoethylenglykole, Dextran oder ein Gemisch davon. Besonders bevorzugt enthält die Oberfläche des Funktionselement-Trägers eine Ethylenoxid-Schicht.

Die vorliegende Erfindung betrifft ebenfalls ein Verfahren zur Herstellung eines erfindungsgemäßen Funktionstestes, wobei auf die Oberfläche eines geeigneten Trägers mindestens eine Schicht eines Verbindungsmittels und danach mindestens eine Mikrostruktur bestehend aus Nanopartikeln mit molekülspezifischen Erkennungssequenzen aufgetragen wird. Erfindungsgemäß ist vorgesehen, dass die Oberfläche eines Funktionselementes vor dem Auftragen der Verbindungsmittel-Schicht vorstrukturiert wird. Nach der Vorstrukturierung der Trägeroberfläche kann dann eine Schicht einer Verbindung auf der vorstrukturierten Trägeroberfläche ausgebracht werden, die eine unspezifische Anlagerung von biologischen Molekülen an der Trägeroberfläche verhindert. Vorzugsweise handelt es sich dabei um eine Ethylenoxid-Schicht.

In bevorzugter Ausführungsform der Erfindung ist vorgesehen, dass die Oberfläche des Trägers des erfindungsgemäßen Funktionselementes nach der Vorstrukturierung und vor dem Ausbringen der Verbindungsmittel-Schicht aktiviert wird. Erfindungsgemäß kann die Aktivierung auch eine Reinigung umfassen. Die Aktivierung der Oberfläche des Trägers des Funktionselementes kann erfindungsgemäß unter Verwendung eines chemischen Verfahrens, insbesondere unter Verwendung von Primern oder Säuren beziehungsweise Basen, erfolgen. Erfindungsgemäß besteht aber auch die Möglichkeit, die Oberfläche des Trägers unter Verwendung eines Plasmas zu aktivieren. Die Aktivierung kann auch das Aufbringen eines Self-Assembled-Monolayers umfassen.

Zur erfindungsgemäßen Erzeugung der Mikrostrukturen auf der Trägeroberfläche des Funktionselementes lassen sich prinzipiell die folgenden zwei Ausführungsformen einsetzen.

In der ersten Ausführungsform des erfindungsgemäßen Verfahrens zur Herstellung eines erfindungsgemäßen Funktionselementes ist vorgesehen, zunächst das Verbindungsmittel strukturiert auf die Oberfläche des Trägers aufzutragen. "Strukturiert" bedeutet im Kontext der Erfindung, dass eine bezüglich Form und Fläche definierte Verbindungsmittel-Schicht auf die Trägeroberfläche aufgetragen wird, wobei die so aufgetragene Verbindungsmittel-Schicht den später von der Mikrostruktur abzudeckenden Flächenabschnitt der Trägeroberfläche definiert. Die Mikrostruktur wird dann durch Eintauchen des Funktionselement-Trägers in eine Nanopartikel-Suspension aufgetragen, wobei die Nanopartikel nur an der strukturiert aufgebrachten Verbindungsmittel-Schicht haften bleiben, nicht jedoch an den Flächenabschnitten der Trägeroberfläche, die keine Verbindungsmittel-Schicht aufweisen. Auf diese Weise wird eine bezüglich Form und Fläche definierte Mikrostruktur erzeugt.

Erfindungsgemäß ist vorgesehen, dass die strukturierte Verbindungsmittel-Schicht beispielsweise mittels eines Nadel-Ring-Printers, eines Tintenstrahlverfahrens, beispielsweise eines Piezo- oder Thermo-Verfahrens, oder eines Mikrokontaktdruckverfahrens aufgetragen wird. Bei Verwendung eines lithografischen Verfahrens, insbesondere des Photolithographie- oder des Mikropen-Lithographie-Verfahrens wird die Trägeroberfläche mit dem Verbindungsmittel bedeckt und dann wird die so erzeugte Verbindungsmittel-Schicht mittels des lithographischen Verfahrens strukturiert. Durch eine geeignete Wahl des Verbindungsmittels kann die aufzutragende Mikrostruktur so gestaltet werden, dass sich die Mikrostruktur oder Teile davon von außen schaltbar, beispielsweise mittels Änderung des pH-Wertes, der Ionenkonzentration oder der Temperatur, zu einem späteren Zeitpunkt wieder lösen lässt/lassen (debond on command). Damit lässt sich beispielsweise eine Mikrostruktur von einem Funktionselement auf ein anderes übertragen.

Stabile Nanopartikel-Suspensionen lassen sich durch Suspendieren der Nanopartikel in Flüssigkeiten, insbesondere wässrigen Medien, gegebenenfalls unter Verwendung zusätzlicher Bestandteile, beispielsweise pH-Mittel, Suspendierhilfen etc., einfach herstellen.

In der zweiten Ausführungsform des erfindungsgemäßen Verfahrens zur Herstellung eines Funktionselementes ist vorgesehen, zunächst den Träger mit einer die gesamte Trägeroberfläche abdeckenden Verbindungsmittel-Schicht zu versehen. Dies kann beispielsweise durch Eintauchen des Trägers in eine Suspension oder Lösung des Verbindungsmittels erfolgen. Anschließend wird die Mikrostruktur dadurch erzeugt, dass eine Nanopartikelsuspension beispielsweise unter Verwendung eines Nadel-Ring-Printers, eines Tintenstrahlverfahrens, zum Beispiel eines Piezo- oder Thermo-Verfahrens, oder eines Mikrokontaktdruckverfahrens strukturiert aufgetragen und somit eine bezüglich Form und Fläche definierte Mikrostruktur erzeugt wird. Bei Verwendung eines lithografischen Verfahrens, insbesondere des Photolithographie- oder des Mikropen-Lithographie-Verfahrens, wird die Trägeroberfläche mit der Nanopartikelsuspension bedeckt und dann wird die so erzeugte Nanopartikelschicht mittels des lithographischen Verfahrens strukturiert.

Bei den zur Erzeugung von Mikrostrukturen auf der Trägeroberfläche eines erfindungsgemäßen Funktionselementes aufgetragenen Nanopartikeln kann es sich um biofunktionalisierbare Nanopartikel handeln, also Nanopartikel, die lediglich molekülspezifische Erkennungsstellen aufweisen, an die jedoch noch keine biologischen Moleküle gebunden sind. Erfindungsgemäß ist es aber auch möglich, zur Strukturierung der Trägeroberfläche biofunktionalisierte Nanopartikel zu verwenden, das heißt Nanopartikel, an deren molekülspezifische Erkennungsstellen bereits biologische Moleküle unter Erhalt ihrer biologischen Aktivität immobilisiert wurden. Erfindungsgemäß ist vorgesehen, dass das immobilisierte biologische Molekül insbesondere ein Protein, ein PNA-Molekül oder eine Nucleinsäure ist.

In einer weiteren bevorzugten Ausführungsform der Erfindung ist vorgesehen, das gleiche Verbindungsmittel und/oder die gleichen Nanopartikeln mehrmals aufzutragen, um mehrlagige fest haftende Mikrostrukturen zu erzeugen. Erfindungsgemäß ist es möglich, eines der vorstehend beschriebenen Verfahren bis zu zehnmal zu wiederholen. Die vorstehend beschriebenen Verfahren können aber auch unter Verwendung unterschiedlicher Verbindungsmittel und/oder unterschiedlicher Nanopartikel wiederholt werden, um Funktionselemente mit unterschiedlichen Mikrostrukturen, die unterschiedliche Funktionen aufweisen, herzustellen.

In einer weiteren bevorzugten Ausführungsform der Erfindung ist vorgesehen, superparamagnetische oder ferromagnetische Eisenoxid-Nanopartikel unter Verwendung eines Magnetfeldes auf einer Trägeroberfläche strukturiert anzuordnen und auf diese Weise direkt Mikrostrukturen, insbesondere nanoskopische Leiterbahnen aufzubauen.

Nach dem Aufbringen der Nanopartikel auf die Trägeroberfläche des Funktionselementes besteht erfindungsgemäß die Möglichkeit, die Partikel anschließend weiter umzusetzen. Enthalten die Partikel beispielsweise Reaktivester, so können diese zur direkten Bindung von Proteinen verwendet werden. Die Nanopartikel können aber auch umgesetzt werden, um sie mit zusätzlichen Funktionen zu versehen. Erfindungsgemäß besteht auch die Möglichkeit, die aus Nanopartikeln bestehende Mikrostrukturen zusätzlich zu fixieren, indem die Partikel beispielsweise untereinander und/oder mit dem Verbindungsmittel kovalent quervernetzt werden.

Die vorliegende Erfindung betrifft auch die Verwendung des erfindungsgemäßen Funktionselementes zur Untersuchung eines Analyten in einer Probe und/oder zu dessen Isolierung und/oder Aufreinigung daraus, wobei das erfindungsgemäße Funktionselement beispielsweise als Gen-Array oder Gen-Chip oder als Protein-Array ausgeführt ist. Im Zusammenhang mit der vorliegenden Erfindung wird unter einem "Analyten" eine Substanz verstanden, bei der Art und Menge ihrer Einzelbestandteile bestimmt und/oder die aus Gemischen abgetrennt werden soll. Insbesondere handelt es sich bei dem Analyten um Proteine, Nucleinsäure, Kohlenhydrate und ähnliche. In bevorzugter Ausführungsform der Erfindung ist der Analyt ein Protein, Peptid, Wirkstoff, Schadstoff, Toxin, Pestizid, Antigen oder eine Nucleinsäure. Unter einer "Probe" wird eine wässrige oder organische Lösung, Emulsion, Dispersion oder Suspension verstanden, die einen vorstehend definierten Analyten in isolierter und aufgereinigter Form oder als Bestandteil eines komplexen Gemisches unterschiedlicher Substanzen enthält. Bei einer Probe kann es sich beispielsweise um eine biologische Flüssigkeit, wie Blut, Lymphe, Gewebeflüssigkeit etc., handeln, also eine Flüssigkeit, die einem lebenden oder toten Organismus, Organ oder Gewebe entnommen wurde. Eine Probe kann jedoch auch ein Kulturmedium, beispielsweise ein Fermentationsmedium, sein, in dem Organismen, beispielsweise Mikroorganismen, oder menschliche, tierische oder pflanzliche Zellen kultiviert wurden. Bei einer Probe im Sinne der Erfindung kann es sich jedoch auch um eine wässrige Lösung, Emulsion, Dispersion oder Suspension eines isolierten und aufgereinigten Analyten handeln. Eine Probe kann bereits Aufreinigungsschritten unterworfen worden sein, kann aber auch ungereinigt vorliegen.

Die vorliegende Erfindung betrifft daher auch die Verwendung des erfindungsgemäßen Funktionselementes zur Durchführung von Analyse- und/oder Detektionsverfahren, wobei es sich bei diesen Verfahren um MALDI-Massenspektroskopie, Fluoreszenz- oder UV-VIS-Spektroskopie, Fluoreszenz- oder Lichtmikroskopie, Wellenleiterspektroskopie oder ein elektrisches Verfahren wie Impedanzspektroskopie handelt. Die vorliegende Erfindung betrifft auch die Verwendung eines erfindungsgemäßen Funktionselementes zur Steuerung der Zelladhäsion oder des Zellwachstums.

Die vorliegende Erfindung betrifft ebenfalls die Verwendung eines erfindungsgemäßen Funktionselementes zum Nachweis und/oder Isolierung biologischer Moleküle. Beispielsweise kann ein erfindungsgemäßes Funktionselement, an dessen Mikrostrukturen eine, vorzugsweise einzelsträngige Nucleinsäure immobilisiert ist, zum Nachweis einer komplementären Nucleinsäure in einer Probe und/oder zur Isolierung dieser komplementären Nucleinsäure eingesetzt werden. Ein erfindungsgemäßes Funktionselement, an dessen Mikrostrukturen ein Protein immobilisiert ist, kann beispielsweise zum Nachweis und/oder zur Isolierung eines mit dem immobilisierten Protein in Wechselwirkung tretenden Proteins aus einer Probe eingesetzt werden.

Die vorliegende Erfindung betrifft auch die Verwendung eines erfindungsgemäßen Funktionselementes zur Entwicklung von pharmazeutischen Präparaten. Die Erfindung betrifft ebenfalls die Verwendung der erfindungsgemäßen Funktionselemente zur Untersuchung der Wirkungen und/oder Nebenwirkungen von pharmazeutischen Präparaten. Die erfindungsgemäßen Funktionselemente lassen sich ebenfalls zur Diagnose von Krankheiten, beispielsweise zur Identifizierung von Krankheitserregern und zur Identifizierung von mutierten Genen, die zur Entstehung von Krankheiten führen, verwenden. Eine weitere Verwendungsmöglichkeit der erfindungsgemäßen Funktionselemente besteht bei der Untersuchung von mikrobiologischen Kontaminationen von Oberflächengewässern, Grundwasser und Böden. Ebenso lassen sich die erfindungsgemäßen Funktionselemente zur Untersuchung der mikrobiologischen Kontamination von Nahrungsmitteln beziehungsweise Tierfutter einsetzen.

Eine weitere bevorzugte Verwendung der erfindungsgemäßen Funktionselemente besteht im Einsatz des erfindungsgemäßen Funktionselementes als Elektronikbaustein, beispielsweise als molekularer Schaltkreis etc., in der Medizintechnik oder in einem Biocomputer. Besonders bevorzugt ist die Verwendung des erfindungsgemäßen Funktionselementes als optischer Speicher in der optischen Informationsverarbeitung, wobei das erfindungsgemäße Funktionselement insbesondere an Mikrostrukturen immobilisierte Photorezeptor-Proteine umfasst, die Licht direkt in ein Signal umwandeln können.

Weitere vorteilhafte Ausgestaltungen der Erfindung ergeben sich aus den Unteransprüchen.

Die Erfindung wird anhand der folgenden Figuren und Beispiele näher erläutert.
Figur 1 zeigt eine lichtmikroskopische Aufnahme von mikrostrukturierten Mikrospots mit einem Durchmesser von 150 µm bis 155 µm aus Nanopartikeln auf einem Siliciumträger.
Figur 2 zeigt eine rasterkraftmikroskopische 3-D-Aufnahme des Randes eines Mikrospots einer 10-fach aufgetragener Nanopartikelsuspension.
Figur 3 zeigt eine lichtmikroskopische Aufnahme von mikrostrukturierten Mikrospots mit einem Durchmesser von 140 µm bis 145 µm aus Nanopartikeln auf einem Glasträger.
Figur 4 zeigt eine lichtmikroskopische Aufnahme von mikrostrukturierten Mikrospots mit einem Durchmesser von 160 µm bis 166 µm aus Nanopartikeln auf einem Glasträger.
Figur 5 zeigt in einem Graphen (Wellenleiterspektroskop) die erhalten gebliebene Funktion von immobilisierten Nanopartikeln. Die Basislinie ist jeweils 0,1 M Phosphatpuffer. A) Zugabe von 0,02 M PDADMAC in 0,1 M NaCl. B) Zugabe von 0,01 M SPS in 0,1 M NaCl. C) Zugabe von 0,5 µM (1) und 1 µM (2) Streptavidin als Kontrollversuch. Dabei ist keine unspezifische Bindung zu erkennen. D) Zugabe von 0,5 % (w/v) der Nanopartikel. E) Bindung von Streptavidin 0,5 - 3 µM. F) Differenz in arcsec. Dies ist die Menge, die spezifisch an die Partikel gebunden wurde.
Figur 6 zeigt eine lichtmikroskopische Differentiale-Interferenz-Kontrast-Aufnahme von Mikrostrukturen. Auf den dunklen Bereichen (Stege) sind die Nanopartikel lokalisiert. Die Stegweite liegt zwischen 10 µm und 13 µm.
Figur 7 zeigt eine rasterkraftmikroskopische Aufnahme (5 x 5 µm²) von Mikrostrukturen.
Figur 8 zeigt die Ergebnisse einer MALDI-TOF-Massenspektroskopie-Analyse unter Verwendung eines Probenträgers, der Monolagen von Protein-beschichteten Nanopartikeln (SPS-PDADMAC-Streptavidin-modifizierte Partikel) aufwies.
Figur 9 zeigt in (A) die Hybridisierung verschiedener Oligonucleotide nach Bindung an einen handelsüblichen Chip mit jeweils einer komplementären fluoreszenzmarkierten DNA und in (B) die Hybridisierung der gleichen Oligonucleotide nach Bindung an eine erfindungsgemäße nanopartikuläre Chip-Oberfläche mit jeweils einer komplementären fluoreszenzmarkierten DNA. Der Vergleich beider Chip-Oberflächen zeigt, dass die erfindungsgemäße Modifikation der Oberfläche mit Nanopartikeln zu einer deutlichen Steigerung der Signalintensität führt.

### Beispiel 1

### Synthese von Silica-Partikeln

Zu 200 ml Ethanol werden 12 mMol Tetraethoxysilan und 90 mMol NH₃ gegeben. Dann wird 24 h bei Raumtemperatur gerührt. Anschließend werden die Partikel durch mehrfache Zentrifugation gereinigt.

Es resultieren 650 mg Silica-Partikel mit einer mittleren Partikelgröße von 125 nm.

### Beispiel 2

### Synthese magnetischer Eisenoxid-Partikel

20 ml einer 1 M FeCl₃-Lösung und 5 ml einer 2 M Fe-SO₄-Lösung in 2 M HCl werden unter kräftigem Rühren zu 250 ml einer 0,7 M NH₃-Lösung gegeben. Dann wird 30 min nachgerührt und der schwarze Feststoff mit 200 ml Wasser gewaschen. Anschließend wird der Niederschlag 30 min mit 100 ml 2 M HNO₃ gerührt und 3-mal mit 100 ml Wasser gewaschen. Die superparamagnetischen Eisenoxid-Nanopartikel werden in 50 ml einer 0,1 M Tetramethylammoniumhydroxid-Lösung resuspendiert.

Es resultieren 2 g Eisenoxid-Partikel mit einer mittleren Partikelgröße von 10 nm.

### Beispiel 3

### Synthese magnetischer Komposit-Partikel

50 mg der vorstehend erhaltenen magnetischen Eisenoxid-Nanopartikel werden zweimal mit 5 ml Ethanol gewaschen und dann in 200 ml Ethanol aufgenommen. Dann werden 12 mMol Tetraethoxysilan und 90 mMol NH₃ zugegeben. Nach 24-stündigem Rühren bei Raumtemperatur werden die die Partikel durch mehrfache Zentrifugation gereinigt.

Es resultieren 600 mg magnetische Komposit-Partikel mit einer mittleren Partikelgröße von 150 nm.

### Beispiel 4

### Synthese fluoreszierender Partikel

190 µMol Fluoresceinamin und 170 µMol Isocyanatopropyltriethoxysilan in 50 ml Ethanol werden 3 h unter Rückfluss gekocht. Zu 50 ml Ethanol werden 3 mMol Tetraethoxysilan und 880 µl der Silan-Farbstoff-Lösung gegeben. Nach Zugabe von 22.5 mMol NH₃ wird 24 h bei Raumtemperatur gerührt. Anschließend werden die Partikel durch mehrfache Zentrifugation gereinigt.

Es resultieren 160 mg Silica-Partikel mit einer mittleren Partikelgröße von 110 nm.

### Beispiel 5

### Synthese organischer Polymer-Nanopartikel

50 mg des Emulgators p-(11-Acrylamido)-undecenoyl-oxyphenyl-dimethylsulfonium-methylsulfat werden in 30 mL Wasser unter Rühren gelöst. Durch diese Lösung wird eine Stunde lang Argon geleitet. Daraufhin erfolgt unter Rühren die Zugabe von 1,8 mL Methylmethacrylat. Die entstandene Emulsion wird auf 60°C erwärmt. Durch Zugabe von 10mg 2,2'-Azobis (2-Amidinopropan) dihydrochlorid wird die Polymerisation gestartet. Nach 5 h wird die Partikelsuspension abgekühlt und die Partikel durch Zentrifugation gereinigt.

Man erhält 1,6 g Partikel mit einer mittleren Partikelgröße von 145 nm. Die Partikel tragen kovalent verknüpfte Sulfonium-Gruppen auf ihrer Oberfläche (Zetapotential im Phosphat-Puffer pH 7,0: + 22 mV) und sind zur Anbindung von Nukleophilen befähigt.

### Beispiel 6

### Oberflächenmodifizierung von Partikeln (aminofunktionalisierte Oberfläche)

Eine 1 Gew.-% wäßrige Suspension der in einem der Beispiele 1 bis 4 erhaltenen Partikel wird mit 10 Vol.-% 25 % Ammoniak versetzt. Dann werden 20 Gew.% Aminpropyltriethoxysilan, bezogen auf die Partikel, zugegeben und es wird 1 h bei Raumtemperatur gerührt. Die Partikel werden durch mehrfache Zentrifugation gereinigt und tragen funktionelle Aminogruppen auf ihrer Oberfläche (Zetapotential im 0,1 M Acetat-Puffer: + 35 mV).

### Beispiel 7

### Oberflächenmodifizierung von Partikeln (aminofunktionalisierte organische Polymerpartikel)

10 mg der in Beispiel 5 erhaltenen Partikel werden in 50 µl eines 10 mMol Phosphatpuffers (pH 7,8) aufgenommen und mit 950 µl einer 1 M Ethylendiamin-Lösung in 10 mMol Phosphatpuffers (pH 7,8) versetzt. Dann wird 2 h bei RT geschüttelt. Danach erfolgt eine Reinigung mittels Zentrifugation. Die Partikel tragen kovalent gebundene Amino-Gruppen auf ihrer Oberfläche.

### Beispiel 8

### Oberflächenmodifizierung von Partikeln (carboxyfunktionalisierte Oberfläche)

10 ml einer 2 Gew.-% Suspension aminofunktionalisierter Nanopartikel werden in Tetrahydrofuran aufgenommen. Dazu werden 260 mg Bernsteinsäureanhydrid gegeben. Nach einer 5-minütigen Behandlung mit Ultraschall wird 1 h bei RT gerührt. Dann werden die Partikel durch mehrfache Zentrifugation gereinigt. Die resultierenden Silica-Partikel tragen funktionelle Carboxygruppen (Zetapotential im 0,1 M Acetat-Puffer von - 35 mV) auf ihrer Oberfläche und besitzen eine mittlere Partikelgröße von 170 nm.

### Beispiel 9

### Oberflächenmodifizierung von Partikeln (Carboxydextran-modifizierte Partikel)

10 mg aminofunktionalisierte Nanopartikel und 1 mg Carboxydextran (Sigma, > 55 cps) werden in 1 ml 0,1 M Morpholinoethansulfonsäure-Puffer (MES, pH: 5,0) vorgelegt. Dazu werden 30 µl einer N-Ethyl-N'-(3-dimethylaminopropyl)-carbodiimid (EDC)-Lösung mit einer Konzentration von 500 µMol/ml zugegeben. Dann wird 30 min bei Raumtemperatur geschüttelt. Dieie Partikel werden abwechselnd mit MES- und TBE-Puffer (89 mM Tris-(hydroxymethyl)aminomethane, 89 mM Borsäure, 2 mM Ethylendiamintetraessigsäure, pH: 8,3) gewaschen und in 1 ml MES-Puffer aufgenommen. Die resultierenden Silica-Partikel tragen funktionelle Carboxygruppen (Zetapotential im 0,1 M Acetat-Puffer von - 25 mV) auf ihrer Oberfläche und besitzen eine mittlere Partikelgröße von 160 nm.

Diese Dextran-Oberfläche ist insbesondere für die Immobilisierung von Proteinen geeignet, deren Tertiär-Struktur durch Adsorptionsprozesse auf der Partikeloberfläche gestört wird.

### Beispiel 10

### Oberflächenmodifizierung von Partikeln (Aminofunktionalisierung der Carboxy-(Dextran)-Partikel)

Man stellt eine 1 M Ethylendiamin-Lösung in 0,1 M MES-Puffer (pH 5,0) her. Dazu werden 500 µg der Carboxy-(Dextran)-modifizierten Partikel und 30 µl einer 500 µMol/ml EDC-Lösung in MES-Puffer gegeben. Dann wird 3 h bei Raumtemperatur (RT) geschüttelt. Anschließend wird mehrmals mit MES-Puffer gewaschen. Es resultieren Partikel mit einer mittleren Partikelgröße von 160 nm und einem Zetapotential von + 25 mV im 0,1 M Acetat-Puffer.

Zur Variation der Spacerlänge bzw. der Dichte der funktionellen Gruppen können auch andere Amine analog eingesetzt werden, beispielsweise 4,7,10-Trioxa-1,13-tridecandiamin (oder höhere Homologe) oder Tris-(2-aminoethyl)-amin.

### Beispiel 11

### Oberflächenmodifizierung von Partikeln (Nitrilotriessigsäure (NTA)-Oberfläche)

10 mg Carboxy-modifizierte Partikel werden 2 mal mit 1 ml Acetonitril (MeCN) gewaschen und in 1 ml MeCN aufgenommen. Dazu werden 10 µMol Dicyclohexylcarbodiimid und 10 µMol N-Hydroxysuccinimid gegeben. Dann wird 2 h bei RT geschüttelt. Danach wird einmal mit 1 ml Cyclohexan und einmal mit 1 ml MeCN gewaschen. Das Reaktionsgemisch wird in 1 ml MeCN aufgenommen. Hierzu werden 4 µMol N,N-Bis-Carboxymethyl-L-Lysin gegeben. Nach 3-stündigem Schütteln bei RT wird einmal mit 1 ml Acetonitril und zweimal mit 1 ml 10 mM Phosphatpuffer (pH 7,0) gewaschen.

Durch diese Umsetzung wird einerseits die Dichte der funktionellen Carboxy-Gruppen erhöht, andererseits können mit dieser Oberfläche Ni²⁺-Ionen durch Komplexierung gebunden werden. Die Oberfläche ist dann zur Bindung His-Tag-modifizierter Proteine befähigt.

### Beispiel 12

### Oberflächenmodifizierung von Partikeln (Thiol-Oberfläche)

10 mg Carboxy-modifizierte Partikel werden 2 mal mit 1 ml Acetonitril (MeCN) gewaschen und in 1 ml MeCN aufgenommen. Hierzu gibt man 10 µMol Dicyclohexylcarbodiimid und 10 µMol N-Hydroxysuccinimid und schüttelt 2 h bei RT. Man wäscht einmal mit 1 ml Cyclohexan und einmal mit 1 ml MeCN und nimmt in 1 ml MeCN auf. Hierzu gibt man 500 µg Cystein und schüttelt 3 h bei RT. Man wäscht einmal mit 1 ml Acetonitril und zweimal mit 1 ml 10 mM Phosphatpuffer (pH 7,0).

Diese Oberfläche ist zur Immobilisierung von Proteinen über Disulfid-Brücken geeignet.

### Beispiel 13

### Funktionalisierung zur Proteinimmobilisierung (Maleimido-aktivierte Oberfläche)

500 µg aminofunktionalisierter Partikel werden in 1 ml 10 mM Phosphatpuffer (pH 7,0) resuspendiert. Dazu werden 1,25 µMol Sulfo-Succinimidyl-4-(N-maleimidomethyl) cyclohexane-1-carboxylat gegeben. Nach 1-stündigem Schütteln bei RT wird einmal mit kaltem 10 mM Phosphatpuffer (pH 7,0) gewaschen und das Gemisch wird in 1 ml 0,1 M Phosphatpuffer (pH 7,0) aufgenommen.

### Beispiel 14

### Funktionalisierung zur Proteinimmobilisierung (Iodacetyl-aktivierte Oberfläche)

500 µg aminofunktionalisierter Partikel werden in 1 ml 10 mM Phosphatpuffer (pH 7,0) resuspendiert. Hierzu gibt man 1,25 µMol Succinimidyl-(4-Iodoacetyl) Aminobenzoate, schüttelt 1 h bei RT, wäscht einmal mit kalter 0,1 M Phosphatpuffer (pH 7,0) und nimmt in 1 ml 10 mM Phosphatpuffer (pH 7,0) auf.

Diese Oberflächen sind zur Ankopplung von Proteinen geeignet, die freie Thiol-Gruppen tragen.

### Beispiel 15

### Funktionalisierung zur Proteinimmobilisierung (biotinylierte Oberfläche)

500 µg aminofunktionalisierter Partikel werden in 1 ml 10 mM Phosphatpuffer (pH 7.0) resuspendiert. Hierzu gibt man 1,25 µMol Succinimidobiotin, schüttelt 1 h bei RT, wäscht einmal mit 0,1 M Phosphatpuffer (pH 7,0) und nimmt in 1 ml 10 mM Phosphatpuffer (pH 7,0) auf.

Die Belegung mit funktionellen Gruppen ist in den Beispielen 13 bis 15 so beschrieben, dass sie quantitativ verläuft. Diese Belegungen lassen sich üblicherweise aber auch durch die Wahl der Reaktionsbedingungen (meist über die Konzentration des Modifikators) so steuern, dass sie nur teilweise erfolgen. Durch eine geeignete Wahl der Modifikatoren können auch verschiedene funktionelle Gruppen nebeneinander auf der Partikeloberfläche vorliegen. Beispiele dafür sind:
- -NH₂ neben -COOH:
   In Beispiel 8 wird die Konzentration an Bernsteinsäureanhydrid verringert. Durch geeignete Wahl des Verhältnisses NH_{2/}COOH lässt sich der isoelektrische Punkt des Partikelsystems in einem weiten Bereich variieren (zwischen 8 und 3). Dies kann bei der Umsetzung mit Proteinen ein entscheidender Parameter zur Steuerung der Reaktivität sein (gleichgeladene Systeme stoßen sich ab).
- -SH neben NTA:
   Die Reaktion wird, wie in den Beispielen 11 oder 12 beschrieben, durchgeführt, wobei allerdings ein Gemisch der beiden Modifikatoren eingesetzt wird. Dadurch können His-Tag tragende Proteine in einem ersten Schritt nicht-kovalent ausgerichtet und anschließend kann dieser Zustand durch Ausbildung einer kovalenten Disulfid-Brücke dauerhaft fixiert werden.

### Beispiel 16

### Immobilisierung von Proteinen (Streptavidinmodifizierte Partikel)

Man legt 2,68 nMol Streptavidin in 10 ml 0,1 m MES-Puffer (pH 5,0) vor. Dazu gibt man 5 mg der in Beispiel 8 gewonnenen Partikel. Dazu werden 2 µMol EDC gegeben. Nach 3-stündigem Schütteln bei RT werden die Partikel einmal mit 10 ml MES-Puffer und einmal mit 10 ml Phosphat-Puffer (pH 7,0) gewaschen. Dann werden die Partikel in 10 ml Phosphat-Puffer (pH 7, 0) aufgenommen.

### Beispiel 17

### Immobilisierung von Proteinen (Protein G-modifizierte Partikel)

Man legt 300 µg Protein G in 10 ml 0,1 m Phosphat-Puffer (pH 7,5) vor. Dazu gibt man 5 mg der Partikel aus Beispiel 5 und schüttelt 3 h bei RT. Man wäscht die Partikel anschließend zweimal mit 10 ml Phosphat-Puffer (pH 7,0) und nimmt in 10 ml Phosphat-Puffer (pH 7,0) auf.

Die Beispiele 16 und 17 zeigen examplarisch die Immobilisierung von zwei Proteinen auf zwei unterschiedlichen Wegen. Auf diesen beiden Wegen lassen sich viele unterschiedliche Proteine immobilisieren. Mit den oben beschriebenen Partikeln sind viele weitere Strategien denkbar, beispielsweise die Aktivierung von Carboxy-modifizierten Partikeln mit Succinimiden, die Kopplung von freien Cysteinen an Thiol-/Maleinimid-Oberflächen. An multifunktionelle Partikel lassen sich auch unterschiedliche Proteine parallel koppeln.

Alle in den Beispielen 1-17 beschriebenen Partikel können direkt für eine Mikrostrukturierung eingesetzt werden.

### Beispiel 18

### Herstellung eines Gen- bzw. Protein-Arrays unter Verwendung eines Ring/Pin-Plotters auf einer präparierten Siliciumoberfläche mit Hilfe von Nanopartikeln mit Silica-Kern

Unter Verwendung eines Ring/Pin-Mikro-Arrayers wurde die Nanopartikelsuspension aus Beispiel 1 auf einem Siliciumträger aufgetragen. Die zugeschnittenen Siliciumträger werden für 90 min in einer 2 Vol.-% wässrigen HELLMANEX^{®} Lösung bei 40°C gelagert. Es folgt eine Ultraschallbadbehandlung für 5 min bei RT und ein Abspülen mit entionisiertem Wasser. Nach Trocknung mit Stickstoff wird die Schichtdicke mittels Nullellipsometer bestimmt.

Die Proben werden anschließend in einer 3:1 (v/v) NH₃/H₂O₂ Lösung bei 70°C für 30 min hydroxyliert (NH₃: puriss. p. a., ~ 25 % in Wasser; H₂O₂: zur Analyse, ISO Reag., stabilisiert, entionisiertes Wasser 18 MΩ). Vor der Lagerung in Wasser (maximal 3h) werden die Proben gründlich mit entionisiertem Wasser gespült. Die Substrate werden bei RT in eine Polyelektrolytlösung (0,02 M Polydiallyldimethylammoniumchlorid (PDADMAC), 0,1 M NaCl, MW = 100 - 200 kDa in entionisiertem Wasser) überführt. Nach 20 min erfolgt ein gründliches Spülen mit entionisiertem Wasser. Die Proben werden 5 min im Ultraschallbad bei RT behandelt, gespült und mit Stickstoff getrocknet. Die so präparierten Siliciumträger werden auf Glasobjektträger (ca. 76 x 26 mm) aufgeklebt. 1 ml der 1 Gew.-% wässrigen Nanopartikelsuspensionen werden jeweils 15 s geschüttelt und im Ultraschallbad bei RT dispergiert.

Die Löcher der Wellplates (96 Löcher ä 300 µl, U-Form) werden mit 100 µl Dispersion befüllt. Die Lösungen in den Plates werden abgeklebt und nach 15 min mit dem Ring/Pin-Mikro-Arrayer (GMS 417, Affymetrix, USA) auf die Siliciumträger bei Laborbedingungen übertragen. Durch eine geschickte Programmierung des Ring/Pin-Mikro-Arrayers erhält man regelmäßig angeordnete, scharf voneinander getrennte Mikrospots mit einem Durchmesser von etwa 150 µm bis 200 µm, wobei der Abstand der individuellen Mikrostrukturen wenige Mikrometer beträgt. Eine Analyse der Mikrospots im Lichtmikroskop beziehungsweise und dem Rasterkraftmikroskop ergab überraschenderweise, dass die Nanopartikel bei 10-fach aufgetragener Nanopartikelsuspension auf ein und dieselbe Position (10 Hits per Dot) am Rand der Mikrospots besonders gehäuft vorkamen. Eine gleichmäßige Verteilung der Nanopartikel innerhalb eines Mikrospots erhält man bei einer Auftragung mit 1-2 Hits per Dot.

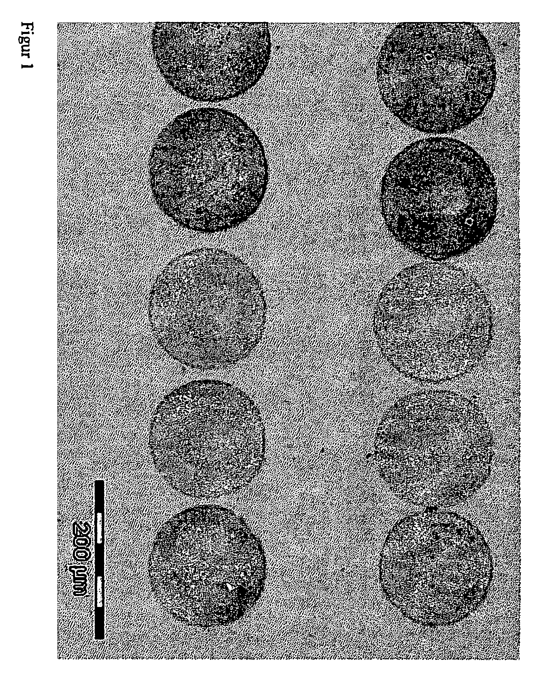

Figur 1 zeigt eine lichtmikroskopische Aufnahme von mikrostrukturierten Mikrospots mit den vorstehend beschriebenen Nanopartikeln mit einem Durchmesser von 150 µm bis 155 µm und einem variablen Abstand von 5 µm bis 20 µm entlang der horizontalen Achse und einem Abstand von 140 µm entlang der vertikalen Achse auf einem Siliciumträger.

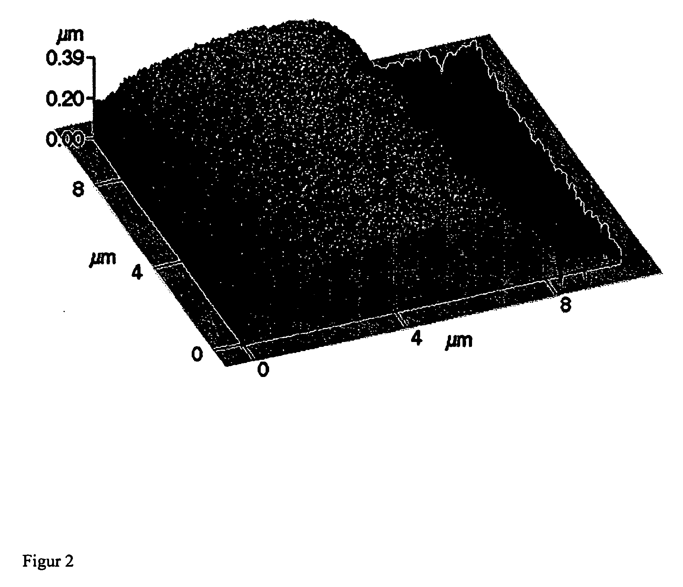

Figur 2 zeigt eine rasterkraftmikroskopische 3-D-Aufnahme des Randes eines Mikrospots einer 10-fach aufgetragener Nanopartikelsuspension auf ein und dieselbe Position, wobei deutlich zu erkennen ist, dass die Nanopartikel im Randbereich des Mikrospots angehäuft vorliegen.

### Beispiel 19

### Herstellung eines Gen- bzw. Protein-Arrays unter Verwendung eines Ring/Pin-Plotters auf einer präparierten Glasoberfläche mit Hilfe von Nanopartikeln mit Silica-Kern

Unter Verwendung eines Ring/Pin-Mikro-Arrayers wurde die Nanopartikelsuspension aus Beispiel 1 auf einen Glasträger aufgetragen. Die Glasträger werden wie in Beispiel 18 vorbehandelt und die 1 Gew.-% wässrige Nanopartikelsuspension wird ebenfalls analog Beispiel 18 auf die Glasoberfläche aufgebracht.

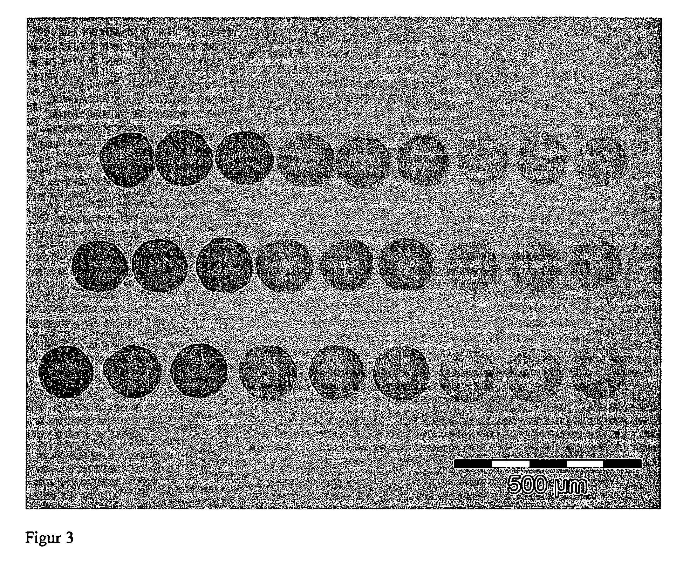

Figur 3 zeigt eine lichtmikroskopische Aufnahme von mikrostrukturierten Mikrospots mit den vorstehend beschriebenen Nanopartikeln mit einem Durchmesser von 140 µm bis 145 µm und einem variablen Abstand von 5 µm bis 20 µm entlang der horizontalen und einem Abstand von 140 µm entlang der vertikalen Achse auf einem Glasträger.

### Beispiel 20

### Herstellung eines Gen- bzw. Protein-Arrays unter Verwendung eines Nadel-Ring-Printers auf einer präparierten Glasoberfläche mit Hilfe von Nanopartikeln mit einem PMMA-Kern

Unter Verwendung eines Ring/Pin-Mikro-Arrayers wurde die Nanopartikelsuspension aus Beispiel 5 auf einem Glasträger aufgetragen. Die Glasträger werden 90 min in einer 2 Vol.-% wässrigen HELLMANEX^{®} Lösung bei 40°C gelagert. Es folgt eine 5-minütige Ultraschallbadbehandlung bei RT und ein Abspülen mit entionisiertem Wasser. Nach Trocknung mit Stickstoff wird die Schichtdicke mittels eines Nullellipsometer bestimmt. Die Proben werden anschließend in einer 3:1 (v/v) NH₃/H₂O₂ Lösung bei 70°C 30 min hydroxyliert (NH₃: puriss. p. a., - 25 % in Wasser; H₂O₂: zur Analyse, ISO Reag., stabilisiert, entionisiertem Wasser 18 MΩ). Vor der Lagerung in Wasser (maximal 3 h) werden die Proben gründlich mit entionisiertem Wasser gespült. Die Substrate werden bei RT in die Polyelektrolytlösung 1 (0,02 M Polydiallyldimethylammoniumchlorid (PDADMAC), 0,1 M NaCl, MW = 100 - 200 kDa in entionisiertem Wasser) überführt. Nach 20 min erfolgt ein gründliches Spülen mit entionisiertem Wasser. Die Glasträger werden dann in die Polyelektrolytlösung 2 (0,01 M Polystyrolsulfonsäure Natriumsalz (SPS), 0,1 M NaCl, MW = 70 kDa in entionisiertem Wasser) für 20 min bei RT überführt. Nach der Lagerung in Polyelektrolytlösung 2 folgt eine 5-minütige Ultraschallbehandlung in entionisiertem Wasser bei RT und die Trocknung mit Stickstoff. Die 1 Gew.-% wässrigen Nanopartikelsuspensionen werden anschließend analog Beispiel 1 und 2 auf die Glasoberfläche aufgebracht.

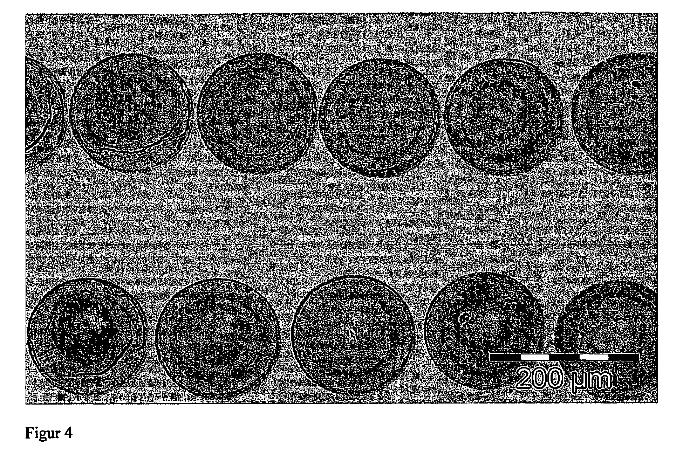

Figur 4 zeigt eine lichtmikroskopische Aufnahme von mikrostrukturierten Mikrospots mit den vorstehend beschriebenen Nanopartikeln mit einem Durchmesser von 160 µm bis 166 µm und einem variablen Abstand von 5 µm bis 20 µm entlang der horizontalen und einem Abstand von 140 µm entlang der vertikalen Achse auf einem Glasträger.

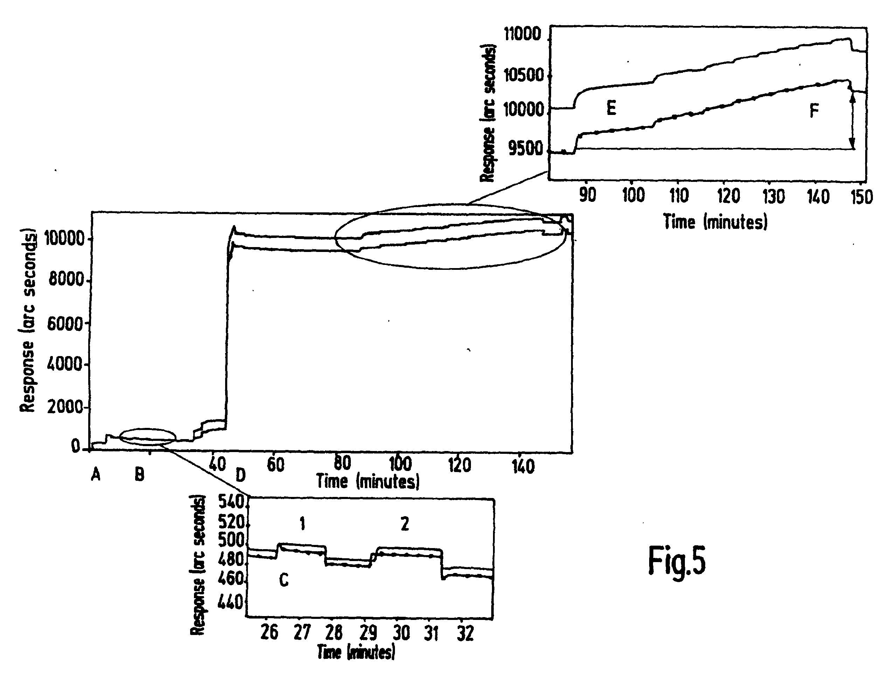

Figur 5 zeigt in einem Graphen (Wellenleiterspektroskop) die erhalten gebliebene Funktion der in Beispiel 5 erhaltenen, immobilisierten Nanopartikeln. Die Basislinie ist jeweils 0,1 M Phosphatpuffer. A) Zugabe von 0,02 M PDADMAC in 0,1 M NaCl. B) Zugabe von 0,01 M SPS in 0,1 M NaCl. C) Zugabe von 0,5 µM (1) und 1 µM (2) Streptavidin als Kontrollversuch. Dabei ist keine unspezifische Bindung zu erkennen. D) Zugabe von 0,5 % (w/v) der Nanopartikel. E) Bindung von Streptavidin 0,5 - 3 µM. F) Differenz in arcsec. Dies ist Menge, die spezifisch an die Partikel gebunden werden konnte.

### Beispiel 21

### Herstellung eines Gen- bzw. Protein-Arrays unter Verwendung eines lithographischen Verfahrens

Siliziumträger, die wie in Beispiel 18 beschrieben beschichtet wurden, werden in einem Abstand von 2 cm unter eine Quecksilber-UV-Lampe (Pen-Ray, UVP, USA) gelegt. Der Steg der Lampe ist um 45° gegenüber der Senkrechten geneigt. Nach Auflegen der Kupfer-Gitter (Plano, Deutschland, d= 3,05 mm) werden die mit PDADMAC beschichteten Träger 45 min bestrahlt. 1 ml der in Beispiel 6 erhaltenen 1 Gew.-% wässrigen Nanopartikelsuspension werden jeweils 15 s geschüttelt und im Ultraschallbad bei Raumtemperatur (RT) dispergiert. Die Kupfergitter werden von dem Träger abgeklopft und es werden jeweils 40 µl der Suspension auf die strukturierte Stelle aufgebracht. Nach 10 min bei Laborbedingungen werden die Proben in entionisiertes Wasser überführt. Danach erfolgt ein 1-minütiges Trocknen mit einem Stickstoff-Strom.

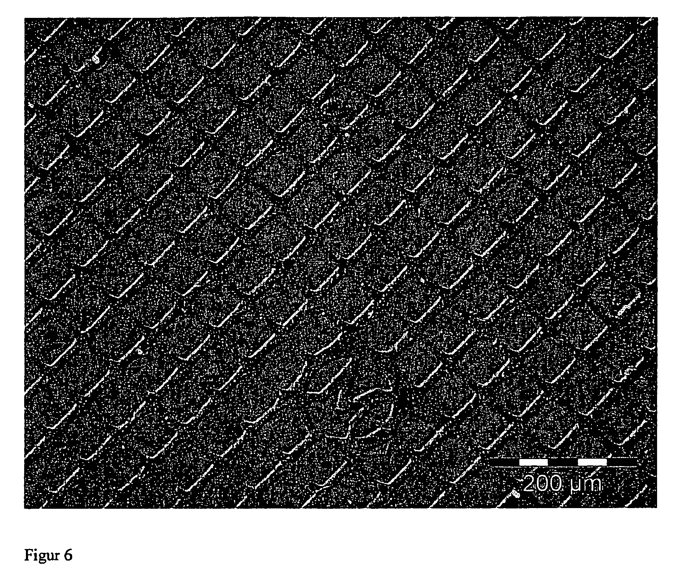

Figur 6 zeigt eine lichtmikroskopische Differentiale-Interferenz-Kontrast-Aufnahme der so erhaltenen Mikrostrukturen. Auf den dunklen Bereichen (Stege) sind die Nanopartikel lokalisiert. Die Stegweite liegt zwischen 10 µm und 13 µm.

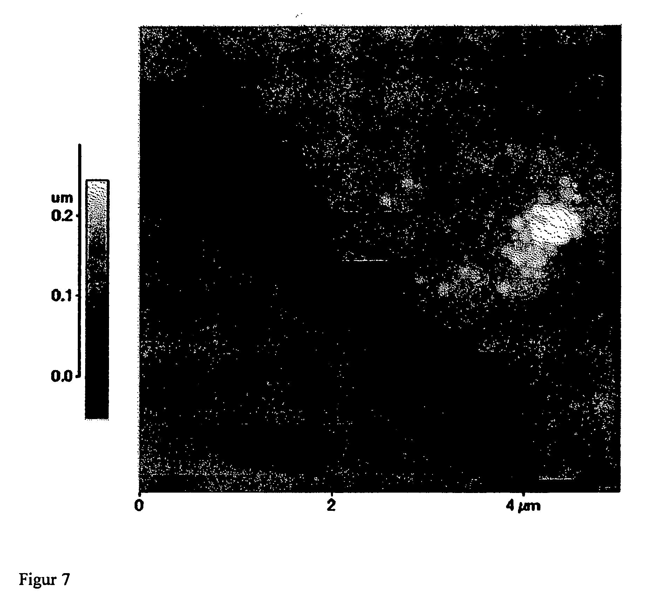

Figur 7 zeigt eine rasterkraftmikroskopische Aufnahme (5 x 5 µm²) der erhaltenen Mikrostrukturen. Die scharfe Abgrenzung zwischen Steg mit Nanopartikeln und unbeschichtetem Substrat ist deutlich zu erkennen.

### Beispiel 22

### MALDI-TOP-Massenspektroskopie-Analyse unter Verwendung eines Probenträgers mit Monolagen aus Protein-beschichteten Nanopartikeln

Dieses Beispiel zeigt, dass Probenträger, die Monolagen von Protein-beschichteten Nanopartikeln aufweisen, direkt in der MALDI-TOF-Massenspektroskopie eingesetzt werden können. Die Analyse wird weder durch die Verbindungsschicht noch durch die Nanopartikel gestört.

### Probenvorbereitung

Die Goldoberfläche des MALDI-Probenträgers wurde zuerst in Aceton, dann in einem 1:1-Gemisch aus Isopropanol (HPLC Grade) und 0,02 N HCl und anschließend in Isopropanol (HPLC-Grade) vorbehandelt (jeweils 10 min im Ultraschallbad). Anschließend erfolgt eine Trocknung mittels Druckluft. Der gereinigte Probenträger wurde dann 20 min in eine SPS-Lösung (0,01 M SPS, 0,1 M NaCl) getaucht, mit entionisiertem Wasser gewaschen und getrocknet. Der Probenträger wurde dann 40 min in eine Suspension der in Beispiel 16 erhaltenen Streptavidinmodifizierten Partikel (0,5 mg/ml) eingetaucht. Die Ladung der Partikel beträgt bei diesem pH-Wert -20 mV. Der Träger wurde mit entionisiertem Wasser gewaschen und getrocknet. Darauf wurden 0,5 µl gesättigter Matrix (3,5-Dimethoxy-4-hydroxy-zimtsäure, gelöst in einem 6:4 (v/v)-Gemisch aus 0,1% Trifluoressigsäure (TFA, Fluka, p.A.) und Acetonitril (Baker, HPLC Grade)) aufgegeben, Luft-getrocknet und danach in einem LD-TOF-MS (HP G 2025A LD-TOF modifiziert mit einer time-lag-focusing (TLF)-Einheit (Future, Fa. GSG); Datenakquise mit Le Croy-500 MHz-Oszilloskop; externe Kalibrierung; Fehler 0,1%) vermessen.

### Ergebnisse

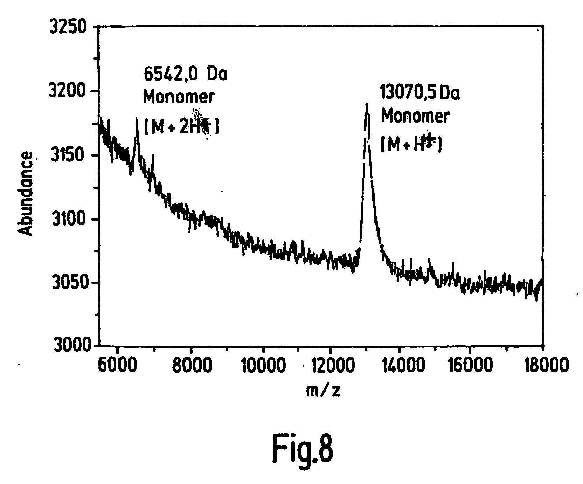

Die Ergebnisse der MALDI-TOF-MS-Analyse sind in Figur 8 gezeigt. Es sind zwei Peaks für Streptavidin zu sehen. Die Peaks bei 13070.5 Da und 6542 Da sind auf das einfach und zweifach geladene Streptavidin-Monomer zurückzuführen. Somit ergibt sich ein Molekulargewicht von 52280 Da für das tetramere Streptavidin. Die Polyelektrolyte PDADMAC und SPS stören nicht die Detektion des Proteins.

### Beispiel 23

### Herstellung eines DNA-Chips für Hybridisierungen und Festphasen-Enzymreaktionen zum Nachweis von Punktmutationen und SNPs sowie für Transkriptions-Untersuchungen

Die in Beispiel 6 erhaltenen Partikel wurden, wie in Beispiel 18 beschrieben, unter Verwendung des Layer-by-Layer-Verfahrens auf eine planare Chip-Oberfläche aufgebracht. Auf die so vorbereiteten Nanopartikel-Träger wurden dann unter Verwendung eines Mikro-Arrayers, also einer Vorrichtung zur Erzeugung von Mikro-Arrays (Pin-and-Ring beziehungsweise Kapillar-Pins), DNA-Sonden aufgetragen. Zum Auftragen wurden DNA-Lösungen in 10 mM Tris/Cl, pH 8,0, 40% DMSO (Vol./Vol.), die jeweils 50 µM DNA enthielten, eingesetzt. Fixierung und Nachbehandlung erfolgten, wie in Diehl, Grahlmann, Beier und Hoheisel, Nucleic Acids Res., 29 (2001), 7, e38, beschrieben. Die so hergestellten DNA-Chips wurden anschließend für Hybridisierungen mit fluoreszenzmarkierten, revers-komplementären DNA-Fragmenten und für Enzym-Reaktionen auf dem Chip nach dem APEX-Prinzip (arrayed primer extension; vgl. Pastinen et al., Genome Res., 10 (2000), 1031-1042) verwendet. Zur Kontrolle wurden die gleichen DNA-Sonden entsprechend Standard-Protokollen auf Poly-L-Lysin-beschichtete Glasobjektträger aufgebracht. Die so hergestellten Kontroll-Chips wurden dann ebenfalls für Hybridisierungen mit fluoreszenzmarkierten, revers-komplementären DNA-Fragmenten und für Enzym-Reaktionen eingesetzt.

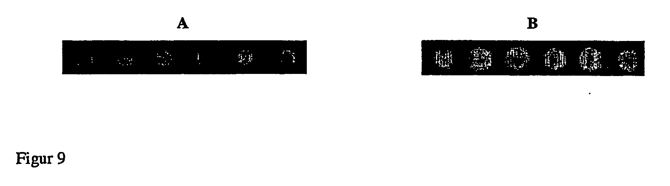

Figur 9 zeigt die Ergebnisse der Hybridisierung unter Verwendung des Kontroll-DNA-Chips im Vergleich zu dem anmeldungsgemäßen nanopartikulären DNA-Chip. Wie aus der Figur zu ersehen ist, wird durch die erfindungsgemäße partikuläre Oberfläche die Signalintensität bei der Hybridisierung signifikant erhöht.

## Patentansprüche

1. Funktionselement, umfassend einen Träger und eine auf der Trägeroberfläche angeordnete adressierbare Mikrostruktur,
wobei die Mikrostruktur aus Nanopartikeln besteht, welche molekülspezifische Erkennungsstellen aufweisen, die ausgewählt sind aus: Proteinen, Nucleinsäuremolekülen, Antikörpern und Komplexen davon sowie molekular geprägten Polymeren und einpolymerisierten Surfmeren, wobei die molekülspezifischen Erkennungsstellen mehrere räumlich fixiert angeordnete erste funktionelle Gruppen aufweisen, woran Moleküle unter Beibehaltung deren biologischer Funktion oder Aktivität an definierten Positionen innerhalb der Moleküle über an die ersten funktionellen Gruppen bindende komplementäre zweite funktionelle Gruppen gerichtet bindbar oder gebunden sind, und
wobei zwischen der Trägeroberfläche und der Mikrostruktur mindestens eine Schicht eines Verbindungsmittels zur festen Anhaftung der Nanopartikel angeordnet ist und das Verbindungsmittel ausgewählt ist aus: geladenen Polymeren, Plasmaschichten mit geladenen Gruppen, Plasmaschichten mit chemisch reaktiven Gruppen und Kombinationen davon.

2. Funktionselement nach Anspruch 1, wobei die Mikrostruktur einen Flächenabschnitt der Trägeroberfläche abdeckt und mindestens einer der Flächen-Längen-Parameter des abgedeckten Flächenabschnitts der Trägeroberfläche kleiner als 999 µm und mindestens 10 nm ist.

3. Funktionselement nach Anspruch 1 oder 2, wobei der Träger und/oder die Oberfläche des Trägers aus einem Metall, Metalloxid, Polymer, Halbleitermaterial, Glas und/oder Keramik besteht.

4. Funktionselement nach einem der Ansprüche 1 bis 3, wobei die Oberfläche des Trägers planar ist.

5. Funktionselement nach einem der Ansprüche 1 bis 3, wobei die Oberfläche des Trägers vorstrukturiert ist.

6. Funktionselement nach einem der Ansprüche 1 bis 5, wobei die Oberfläche des Trägers eine Schicht einer chemischen Verbindung aufweist, die eine unspezifische Anlagerung von biologischen Molekülen an der Trägeroberfläche verhindert.

7. Funktionselement nach einem der Ansprüche 1 bis 6, wobei das als Verbindungsmittel eingesetzte Polymer ein Hydrogel ist.

8. Funktionselement nach einem der Ansprüche 1 bis 7, wobei das Verbindungsmittel durch Änderung des pH-Wertes, der Ionenkonzentration oder der Temperatur schaltbar ist.

9. Funktionselement nach einem der Ansprüche 1 bis 8, wobei die Nanopartikel einen Kern und eine die molekülspezifischen Erkennungsstellen aufweisende Oberfläche umfassen.

10. Funktionselement nach Anspruch 9, wobei ein oder mehrere biologisch aktive Moleküle an den molekülspezifischen Erkennungsstellen gebunden sind.

11. Funktionselement nach Anspruch 10, wobei die biologisch aktiven Moleküle kovalent und/oder nicht-kovalent gebunden sind.

12. Funktionselement nach Anspruch 10 oder 11, wobei die Moleküle unter Erhalt ihrer biologischen Aktivität gebunden sind.

13. Funktionselement nach einem der Ansprüche 10 bis 12, wobei es sich bei den gebundenen Molekülen um Proteine, Nucleinsäuren, PNA-Moleküle oder Fragmente davon handelt.

14. Funktionselement nach Anspruch 13, wobei die Proteine Antikörper, Antigene, Enzyme, Cytokine oder Rezeptoren sind.

15. Funktionselement nach einem der Ansprüche 1 bis 14, wobei die ersten funktionellen Gruppen und die die ersten funktionellen Gruppen bindenden komplementären zweiten funktionellen Gruppen ausgewählt sind aus der Gruppe bestehend aus Aktivester, Alkylketongruppe, Aldehydgruppe, Aminogruppe, Carboxygruppe, Epoxygruppe, Maleinimidogruppe, Hydrazingruppe, Hydrazidgruppe, Thiolgruppe, Thioestergruppe, Oligohistidingruppe, Strep-Tag I, Strep-Tag II, Desthiobiotin, Biotin, Chitin, Chitinderivate, Chitinbindedomäne, Metallchelatkomplex, Streptavidin, Streptactin, Avidin und Neutravidin.

16. Funktionselement nach Anspruch 15, wobei die ersten und die zweiten funktionellen Gruppen durch molekulares Prägen erzeugt sind.

17. Funktionselement nach Anspruch 15 oder 16, wobei die ersten funktionellen Gruppen Bestandteil eines Spacers sind oder über Spacer mit der Oberfläche der Nanopartikel verbunden sind.

18. Funktionselement nach Anspruch 15 oder 16, wobei die komplementären zweiten funktionellen Gruppen Bestandteil eines Spacers sind oder über Spacer mit den Molekülen verbunden sind.

19. Funktionselement nach einem der Ansprüche 9 bis 18, wobei der Kern der Nanopartikel aus einem organischen Material besteht oder dieses enthält.

20. Funktionselement nach Anspruch 19, wobei das organische Material ein organisches Polymer ist.

21. Funktionselement nach Anspruch 19 oder 20, wobei das organische Polymer Polypropylen, Polystyrol, Polyacrylat oder ein Gemisch davon ist.

22. Funktionselement nach einem der Ansprüche 9 bis 18, wobei der Kern aus einem anorganischen Material besteht oder dieses enthält.

23. Funktionselement nach Anspruch 22, wobei das anorganische Material ein Metall wie Au, Ag oder Ni, Silicium, SiO₂, SiO, ein Silicat, Al₂O₃, SiO₂·Al₂O₃, Fe₂O₃, Ag₂O, TiO₂, ZrO₂, Zr₂O₃, Ta₂O₅, Zeolith, Glas, Indiumzinnoxid, Hydroxylapatit, ein Q-Dot oder ein Gemisch davon ist.

24. Funktionselement nach einem der Ansprüche 9 bis 23, wobei der Kern eine Größe von 5 nm bis 500 nm aufweist.

25. Funktionselement nach einem der Ansprüche 9 bis 24, wobei der Kern mindestens eine zusätzliche Funktion aufweist.

26. Funktionselement nach Anspruch 25, wobei die zusätzliche Funktion im Kern verankert ist und eine Fluoreszenzmarkierung, eine UV/Vis-Markierung, eine superparamagnetische Funktion, eine ferromagnetische Funktion und/oder eine radioaktive Markierung ist.

27. Funktionselement nach Anspruch 25, wobei die Oberfläche des Kerns mit einer die ersten funktionellen Gruppen enthaltenden organischen oder anorganischen Schicht modifiziert ist, die eine Fluoreszenzmarkierung, eine UV/Vis-Markierung, eine superparamagnetische Funktion, eine ferromagnetische Funktion und/oder eine radioaktive Markierung aufweist.

28. Funktionselement nach einem der Ansprüche 25 bis 27, wobei die Oberfläche des Kerns eine chemische Verbindung aufweist, die zur sterischen Stabilisierung und/oder zur Verhinderung einer Konformationsänderung der immobilisierten Moleküle und/oder zur Verhinderung der Anlagerung einer weiteren biologisch aktiven Verbindung an den Kern dient.

29. Funktionselement nach Anspruch 28, wobei die chemische Verbindung ein Polyethylenglykol, ein Oligoethylenglykol, Dextran oder ein Gemisch davon ist.

30. Funktionselement nach einem der vorhergehenden Ansprüche, wobei die gebundenen Moleküle einen Marker aufweisen.

31. Funktionselement nach einem der vorhergehenden Ansprüche, wobei an die gebundenen Moleküle weitere Moleküle gebunden sind.

32. Funktionselement nach einem der vorhergehenden Ansprüche, wobei die Mikrostruktur aus einer Nanopartikel-Schicht besteht.

33. Funktionselement nach einem der vorhergehenden Ansprüche, wobei die Mikrostruktur aus mehreren Nanopartikel-Schichten besteht.

34. Funktionselement nach einem der vorhergehenden Ansprüche, wobei auf der Trägeroberfläche mehrere Mikrostrukturen angeordnet sind, die aus Nanopartikeln mit unterschiedlichen molekülspezifischen Erkennungsstellen bestehen.

35. Funktionselement nach Anspruch 34, wobei an den Mikrostrukturen unterschiedliche Moleküle gebunden sind.

36. Funktionselement nach einem der Ansprüche 1 bis 35, erhältlich durch Auftragen einer oder mehrerer Mikrostrukturen auf die Trägeroberfläche unter Verwendung eines Nadel-Ring-Printers.

37. Funktionselement nach einem der Ansprüche 1 bis 35, erhältlich durch Auftragen einer oder mehrerer Mikrostrukturen auf die Trägeroberfläche unter Verwendung eines lithografischen Verfahrens.

38. Funktionselement nach Anspruch 37, wobei das lithographische Verfahren Photolithographie ist.

39. Funktionselement nach Anspruch 37, wobei das lithographische Verfahren Mikropen-Lithographie ist.

40. Funktionselement nach einem der Ansprüche 1 bis 35, erhältlich durch Auftragen einer oder mehrerer Mikrostrukturen auf die Trägeroberfläche unter Verwendung eines Tintenstrahlverfahrens.

41. Funktionselement nach einem der Ansprüche 1 bis 35, erhältlich durch Auftragen einer oder mehrerer Mikrostrukturen unter Verwendung eines Mikrokontaktdruckverfahrens.

42. Verfahren zur Herstellung eines Funktionselementes nach einem der Ansprüche 1 bis 41, wobei auf die Oberfläche eines Trägers mindestens eine Schicht des Verbindungsmittels gemäß Anspruch 1 und danach mindestens eine Mikrostruktur bestehend aus Nanopartikeln mit molekülspezifischen Erkennungsstellen gemäß Anspruch 1 aufgetragen werden.

43. Verfahren nach Anspruch 42, wobei die Oberfläche des Trägers vor dem Auftragen der Verbindungsmittel-Schicht gereinigt und/oder aktiviert wird.

44. Verfahren nach Anspruch 43, wobei die Trägeroberfläche chemisch aktiviert wird.

45. Verfahren nach Anspruch 44, wobei die Trägeroberfläche mit Ladungen versehen wird.

46. Verfahren nach Anspruch 44 oder 45, wobei die Trägeroberfläche durch Aufbringen eines Primers aktiviert wird.

47. Verfahren nach Anspruch 44 oder 45, wobei eine Self-Assembly-Schicht auf die Trägeroberfläche aufgebracht wird.

48. Verfahren nach Anspruch 43, wobei die Trägeroberfläche mittels eines Plasmas aktiviert wird.

49. Verfahren nach einem der Ansprüche 42 bis 48, wobei auf die Trägeroberfläche eine bezüglich Form und Fläche definierte Verbindungsmittel-Schicht aufgebracht wird und der Träger danach in eine Nanopartikel-Suspension eingetaucht wird, so dass durch Anhaften der Nanopartikel an der aufgebrachten Verbindungsmittel-Schicht eine bezüglich Form und Fläche definierte Mikrostruktur erzeugt wird.

50. Verfahren nach Anspruch 49, wobei die bezüglich Form und Fläche definierte Verbindungsmittel-Schicht mittels eines Nadel-Ring-Printers, eines lithografischen Verfahrens, eines Tintenstrahlverfahrens oder eines Mikrokontaktdruckverfahrens aufgetragen wird.

51. Verfahren nach einem der Ansprüche 42 bis 50, wobei der Träger in eine Suspension oder Lösung des Verbindungsmittels eingetaucht wird, so dass eine die gesamte Trägeroberfläche abdeckende Verbindungsmittel-Schicht erzeugt wird, und danach die Nanopartikel so aufgetragen werden, dass eine bezüglich Form und Fläche definierte Mikrostruktur erzeugt wird.

52. Verfahren nach Anspruch 51, wobei die bezüglich Form und Fläche definierte Mikrostruktur mittels eines Nadel-Ring-Printers, eines lithografischen Verfahrens, eines Tintenstrahlverfahrens oder eines Mikrokontaktdruckverfahrens aufgetragen wird.

53. Verfahren nach einem der Ansprüche 42 bis 52, wobei das Verbindungsmittel und die Nanopartikel mehrmals auf die Trägeroberfläche aufgebracht werden.

54. Verfahren nach einem der Ansprüche 42 bis 53, wobei vor dem Aufbringen der Nanopartikel biologisch aktive Moleküle an die molekülspezifischen Erkennungsstellen der Nanopartikel gebunden werden.

55. Verfahren nach einem der Ansprüche 42 bis 53, wobei nach dem Aufbringen der Nanopartikel biologisch aktive Moleküle an die molekülspezifischen Erkennungsstellen der Nanopartikel gebunden werden.

56. Verfahren nach einem der Ansprüche 42 bis 53, wobei vor und nach dem Aufbringen der Nanopartikel biologisch aktive Moleküle an die molekülspezifischen Erkennungsstellen der Nanopartikel gebunden werden.

57. Verfahren nach einem der Ansprüche 54 bis 56, wobei die Bindung der biologisch aktiven Moleküle an die molekülspezifischen Erkennungsstellen der Nanopartikel erfolgt, indem die erste funktionelle Gruppen aufweisenden molekülspezifischen Erkennungsstellen der Nanopartikel mit den die ersten funktionellen Gruppen bindenden, komplementären zweiten funktionellen Gruppen aufweisenden Molekülen derart in Kontakt gebracht werden, dass kovalente und/oder nicht-kovalente Bindungen zwischen den funktionellen Gruppen der molekülspezifischen Erkennungsstellen und der Moleküle erfolgen.

58. Verfahren nach Anspruch 57, wobei die ersten funktionellen Gruppen und die die ersten funktionellen Gruppen bindenden komplementären zweiten funktionellen Gruppen ausgewählt sind aus der Gruppe bestehend aus Aktivester, Alkylketongruppe, Aldehydgruppe, Aminogruppe, Carboxygruppe, Epoxygruppe, Maleinimidogruppe, Hydrazingruppe, Hydrazidgruppe, Thiolgruppe, Thioestergruppe, Oligohistidingruppe, Strep-Tag I, Strep-Tag II, Desthiobiotin, Biotin, Chitin, Chitinderivate, Chitinbindedomäne, Metallchelatkomplex, Streptavidin, Streptactin, Avidin und Neutravidin.

59. Verfahren nach einem der Ansprüche 54 bis 58, wobei die biologisch aktiven Moleküle unter Erhalt ihrer biologischen Aktivität gebunden werden.

60. Verfahren nach einem der Ansprüche 54 bis 59, wobei es sich bei den Molekülen um Proteine, Antigene, Nucleinsäuren, PNA-Moleküle oder Fragmente davon handelt.

61. Verwendung eines Funktionselementes nach einem der Ansprüche 1 bis 41 oder eines Funktionselementes, hergestellt nach einem Verfahren nach einem der Ansprüche 42 bis 60 zur Durchführung eines Detektionsverfahrens.

62. Verwendung nach Anspruch 61, wobei das Detektionsverfahren MALDI-Massenspektroskopie, Fluoreszenz- oder UV-Vis-Spektroskopie, Fluoreszenz- oder Lichtmikroskopie, Wellenleiterspektroskopie, Impedanzspektroskopie oder ein anderes elektrisches Verfahren ist.

63. Verwendung eines Funktionselementes nach einem der Ansprüche 1 bis 41 oder eines Funktionselementes, hergestellt nach einem Verfahren nach einem der Ansprüche 42 bis 60 zur Steuerung der Zelladhäsion oder des Zellwachstums.

64. Verwendung eines Funktionselementes nach einem der Ansprüche 1 bis 41 oder eines Funktionselementes, hergestellt nach einem Verfahren nach einem der Ansprüche 42 bis 60 zur Entwicklung von pharmazeutischen Präparaten.

65. Verwendung eines Funktionselementes nach einem der Ansprüche 1 bis 41 oder eines Funktionselementes hergestellt nach einem Verfahren nach einem der Ansprüche 42 bis 60 zur Analyse der Wirkungen und/oder Nebenwirkungen von pharmazeutischen Präparaten.

66. Verwendung eines Funktionselementes nach einem der Ansprüche 1 bis 41 oder eines Funktionselementes hergestellt nach einem Verfahren nach einem der Ansprüche 42 bis 60 zur Diagnose von Krankheiten.

67. Verwendung nach Anspruch 66, wobei das Funktionselement zur Identifizierung von Krankheitserregern eingesetzt wird.

68. Verwendung nach Anspruch 66, wobei das Funktionselement zur Identifizierung von mutierten Genen bei einem Menschen oder einem Tier eingesetzt wird.

69. Verwendung eines Funktionselementes nach einem der Ansprüche 1 bis 41 oder eines Funktionselements hergestellt nach einem Verfahren nach einem der Ansprüche 42 bis 60 zur Analyse der mikrobiologischen Kontamination von Proben.

70. Verwendung nach Anspruch 69, wobei die Probe eine Wasser- oder Bodenprobe ist.

71. Verwendung nach Anspruch 69, wobei die Probe aus einem Nahrungsmittel oder Tierfutter stammt.

72. Verwendung eines Funktionselementes nach einem der Ansprüche 1 bis 41 oder eines Funktionselementes, hergestellt nach einem Verfahren nach einem der Ansprüche 42 bis 60 als Elektronikbaustein in einem Biocomputer.

## Claims

1. Functional element, comprising a carrier and an addressable microstructure arranged on the carrier surface,
the microstructure consisting of nanoparticles having molecule-specific recognition sites selected from the group consisting of proteins, nucleic acid molecules, antibodies and complexes thereof as well as molecularly imprinted polymers and surfmeres incorporated by polymerization,
the molecule-specific recognition sites having several first functional groups being arranged in a spatially fixed manner to which molecules can be specifically bound to or are specifically bound to via the complementary second functional groups which bind to the first functional groups, the molecules preserving their biologically functioning or activity at defined positions within the molecules, and
at least one layer of a bonding agent being arranged between the carrier surface and the microstructure to ensure permanent adherence of the nanoparticles, the bonding agent being selected from the group consisting of charged polymers, plasma layers with charged groups, plasma layers with chemically reactive groups and combinations thereof.

2. Functional element according to claim 1, wherein the microstructure covers a portion of the carrier surface and at least one of the area/length parameters of the covered portion of the carrier surface is smaller than 999 µm and at least 10 nm.

3. Functional element according to claim 1 or 2, wherein the carrier and/or the surface of the carrier consist/s of a metal, metal oxide, polymer, semiconductor material, glass and/or ceramic.

4. Functional element according to one of the claims 1 to 3, wherein the surface of the carrier is planar.

5. Functional element according to one of the claims 1 to 3, wherein the surface of the carrier is pre-structured.

6. Functional element according to one of the claims 1 to 5, wherein the surface of the carrier has a layer of a chemical compound that prevents nonspecific attachment of biological molecules to the carrier surface.

7. Functional element according to one of the claims 1 to 6, wherein the polymer used as bonding agent is a hydrogel.

8. Functional element according to one of the claims 1 to 7, wherein the bonding agent is switchable by altering the pH value, the ion concentration or the temperature.

9. Functional element according to one of the claims 1 to 8, wherein the nanoparticles comprise a core and a surface that has the molecule-specific recognition sites.

10. Functional element according to claim 9, wherein one or more biologically active molecules are bound to the molecule-specific recognition sites.

11. Functional element according to claim 10, wherein the biologically active molecules are bound covalently and/or non-covalently.

12. Functional element according to claim 10 or 11, wherein the molecules are bound preserving their biological activity.

13. Functional element according to one of the claims 10 to 12, wherein the bound molecules are proteins, nucleic acids, PNA molecules or fragments thereof.

14. Functional element according to claim 13, wherein the proteins are antibodies, antigens, enzymes, cytokines or receptors.

15. Functional element according to one of the claims 1 to 14, wherein the first functional groups and the complementary second functional groups that bind the first functional groups are selected from the group comprising active ester, alkyl ketone group, aldehyde group, amino group, carboxy group, epoxy group, maleinimide group, hydrazine group, hydrazide group, thiol group, thioester group, oligohistidine group, Strep-tag I, Strep-tag II, desthiobiotin, biotin, chitin, chitin derivatives, chitin binding domain, metal chelate complex, streptavidin, streptactin, avidin and neutravidin.

16. Functional element according to claim 15, wherein the first and the second functional groups are produced by molecular imprinting.

17. Functional element according to claim 15 or 16, wherein the first functional groups are a component part of a spacer or are bound via spacers to the surface of the nanoparticles.

18. Functional element according to claim 15 or 16, wherein the complementary second functional groups are a component part of a spacer or are bound via spacers to the molecules.

19. Functional element according to one of the claims 9 to 18, wherein the core of the nanoparticles consists of or contains an organic material.

20. Functional element according to claim 19, wherein the organic material is an organic polymer.

21. Functional element according to claim 19 or 20, wherein the organic polymer is polypropylene, polystyrene, polyacrylate or a mixture thereof.

22. Functional element according to one of the claims 9 to 18, wherein the core consists of or contains an inorganic material.

23. Functional element according to claim 22, wherein the inorganic material is a metal such as Au, Ag or Ni, silicon, SiO₂, SiO, a silicate, Al₂O₃, SiO₂·Al₂O₃, Fe₂O₃, Ag₂O, TiO₂, ZrO₂, Zr₂O₃, Ta₂O₅, zeolite, glass, indium-tin oxide, hydroxylapatite, a Q-Dot or a mixture thereof.

24. Functional element according to one of the claims 9 to 23, wherein the core has a size from 5 nm to 500 nm.

25. Functional element according to one of the claims 9 to 24, wherein the core has at least one additional function.

26. Functional element according to claim 25, wherein the additional function is anchored in the core and is a fluorescence marker, a UV/Vis marker, a superparamagnetic function, a ferromagnetic function and/or a radioactive marker.

27. Functional element according to claim 25, wherein the surface of the core is modified with an organic or inorganic layer containing the first functional groups, which has a fluorescence marker, a UV/Vis marker, a superparamagnetic function, a ferromagnetic function and/or a radioactive marker.

28. Functional element according to one of the claims 25 to 27, wherein the surface of the core has a chemical compound, which serves for steric stabilization and/or for preventing a change of conformation of the immobilized molecules and/or for preventing the attachment of a further biologically active compound to the core.

29. Functional element according to claim 28, wherein the chemical compound is a polyethylene glycol, an oligoethylene glycol, dextran or a mixture thereof.

30. Functional element according to any one of the preceding claims, wherein the bound molecules have a marker.

31. Functional element according to any one of the preceding claims, wherein further molecules are bound to the bound molecules.

32. Functional element according to any one of the preceding claims, wherein the microstructure consists of a nanoparticle layer.

33. Functional element according to any one of the preceding claims, wherein the microstructure consists of several nanoparticle layers.

34. Functional element according to any one of the preceding claims, wherein several microstructures, which consist of nanoparticles with different molecule-specific recognition sites, are arranged on the carrier surface.

35. Functional element according to claim 34, wherein various molecules are bound to the microstructures.

36. Functional element according to one of the claims 1 to 35, obtainable by applying one or more microstructures to the carrier surface using a ring/pin printer.

37. Functional element according to one of the claims 1 to 35, obtainable by applying one or more microstructures to the carrier surface using a lithographic process.

38. Functional element according to claim 37, wherein the lithographic process is photolithography.

39. Functional element according to claim 37, wherein the lithographic process is micropen lithography.

40. Functional element according to one of the claims 1 to 35, obtainable by applying one or more microstructures to the carrier surface using an InkJet process.

41. Functional element according to one of the claims 1 to 35, obtainable by applying one or more microstructures using a microcontact printing process.

42. Method for the production of a functional element according to one of the claims 1 to 41, wherein at least one layer of the bonding agent according to claim 1 and then at least one microstructure consisting of nanoparticles with molecule-specific recognition sites according to claim 1 are applied to the surface of a carrier.

43. Method according to claim 42, wherein the surface of the carrier is cleaned and/or activated before applying the layer of bonding agent.

44. Method according to claim 43, wherein the carrier surface is activated chemically.

45. Method according to claim 44, wherein the carrier surface is provided with charges.

46. Method according to claim 44 or 45, wherein the carrier surface is activated by applying a primer.

47. Method according to claim 44 or 45, wherein a self-assembly layer is applied to the carrier surface.

48. Method according to claim 43, wherein the carrier surface is activated by means of a plasma.

49. Method according to one of the claims 42 to 48, wherein a layer of bonding agent defined with respect to shape and area is applied to the carrier surface and the carrier is then dipped into a nanoparticle suspension, so that a microstructure that is defined with respect to shape and area is produced through adherence of the nanoparticles to the applied layer of bonding agent.

50. Method according to claim 49, wherein the layer of bonding agent defined with respect to shape and area is applied by means of a ring/pin printer, a lithographic process, an InkJet process or a microcontact printing process.

51. Method according to one of the claims 42 to 50, wherein the carrier is dipped into a suspension or solution of the bonding agent, so that a layer of bonding agent covering the whole carrier surface is produced, and then the nanoparticles are applied in such a way that a microstructure defined with respect to shape and area is produced.

52. Method according to claim 51, wherein the microstructure defined with respect to shape and area is applied by means of a ring/pin printer, a lithographic process, an inkjet process or a microcontact printing process.

53. Method according to one of the claims 42 to 52, wherein the bonding agent and the nanoparticles are applied to the carrier surface several times.

54. Method according to one of the claims 42 to 53, wherein biologically active molecules are bound to the molecule-specific recognition sites of the nanoparticles before the nanoparticles are applied.

55. Method according to one of the claims 42 to 53, wherein biologically active molecules are bound to the molecule-specific recognition sites of the nanoparticles after application of the nanoparticles.

56. Method according to one of the claims 42 to 53, wherein biologically active molecules are bound to the molecule-specific recognition sites of the nanoparticles before and after application of the nanoparticles.

57. Method according to one of the claims 54 to 56, wherein the binding of the biologically active molecules to the molecule-specific recognition sites of the nanoparticles is effected by bringing the molecule-specific recognition sites of the nanoparticles, which have first functional groups, into contact with the molecules that have complementary second functional groups that bind the first functional groups, in such a way that covalent and/or non-covalent bonds are effected between the functional groups of the molecule-specific recognition sites and the molecules.

58. Method according to claim 57, wherein the first functional groups and the complementary second functional groups that bind the first functional groups are selected from the group consisting of active ester, alkyl ketone group, aldehyde group, amino group, carboxy group, epoxy group, maleinimide group, hydrazine group, hydrazide group, thiol group, thioester group, oligohistidine group, Strep-tag I, Strep-tag II, desthiobiotin, biotin, chitin, chitin derivatives, chitin binding domain, metal chelate complex, streptavidin, streptactin, avidin and neutravidin.

59. Method according to one of the claims 54 to 58, wherein the biologically active molecules are bound preserving their biological activity.

60. Method according to one of the claims 54 to 59, wherein the molecules are proteins, antigens, nucleic acids, PNA molecules or fragments thereof.

61. Use of a functional element according to one of the claims 1 to 41 or of a functional element produced according to a method of one of claims 42 to 60 for carrying out a detection method.

62. Use according to claim 61, wherein the method of detection is MALDI mass spectroscopy, fluorescence or UV-Vis spectroscopy, fluorescence or light microscopy, waveguide spectroscopy, impedance spectroscopy or some other electrical method.

63. Use of a functional element according to one of the claims 1 to 41 or of a functional element produced according to a method of one of claims 42 to 60 for controlling cellular adhesion or cellular growth.

64. Use of a functional element according to one of the claims 1 to 41 or of a functional element produced according to a method of one of claims 42 to 60 for developing pharmaceutical preparations.

65. Use of a functional element according to one of the claims 1 to 41 or of a functional element produced according to a method of one of claims 42 to 60 for analyzing the effects and/or side effects of pharmaceutical preparations.

66. Use of a functional element according to one of the claims 1 to 41 or of a functional element produced according to a method of one of claims 42 to 60 for diagnosing diseases.

67. Use according to claim 66, wherein the functional element is used for identifying pathogens.

68. Use according to claim 66, wherein the functional element is used for identifying mutated genes in a human being or an animal.

69. Use of a functional element according to one of the claims 1 to 41 or of a functional element produced according to a method of one of claims 42 to 60 for analyzing the microbiological contamination of samples.

70. Use according to claim 69, wherein the sample is a water sample or a soil sample.

71. Use according to claim 69, wherein the sample is obtained from foodstuff or animal feed.

72. Use of a functional element according to one of the claims 1 to 41 or of a functional element produced according to a method of one of claims 42 to 60 as an electronic component in a biocomputer.

## Revendications

1. Elément fonctionnel comprenant un support et une microstructure adressable disposée sur la surface du support,
dans lequel la microstructure se compose de nanoparticules qui présentent des points de reconnaissance spécifiques à une molécule qui sont sélectionnés parmi : des protéines, des molécules d'acide nucléique, des anticorps et des complexes de ceux-ci ainsi que des polymères marqués par voie moléculaire et des surfmères polymérisés,
dans lequel les points de reconnaissance spécifiques à une molécule présentent plusieurs premiers groupes fonctionnels disposés de manière fixe dans l'espace auxquels des molécules peuvent être liées ou sont liées de manière dirigée en conservant leur fonction ou activité biologique en des positions définies au sein des molécules par le biais de seconds groupes fonctionnels fixant les premiers groupes fonctionnels, et
dans lequel au moins une couche d'un moyen de liaison pour l'adhésion fixe des nanoparticules est disposée entre la surface du support et la microstructure et que le moyen de liaison est sélectionné parmi : des polymères chargés, des couches de plasma avec des groupes chargés, des couches de plasma avec des groupes chimiquement réactifs et des combinaisons de ceux-ci.

2. Elément fonctionnel selon la revendication 1, dans lequel la microstructure recouvre une section de surface de la surface du support et au moins un des paramètres de longueur de surface de la section de surface recouverte de la surface du support est inférieur à 999 µm et d'au moins 10 nm.

3. Elément fonctionnel selon la revendication 1 ou 2, dans lequel le support et/ou la surface du support se compose d'un métal, d'un oxyde métallique, d'un polymère, d'un matériau semi-conducteur, de verre et/ou de céramique.

4. Elément fonctionnel selon l'une quelconque des revendications 1 à 3, dans lequel la surface du support est plane.

5. Elément fonctionnel selon l'une quelconque des revendications 1 à 3, dans lequel la surface du support est préstructurée.

6. Elément fonctionnel selon l'une quelconque des revendications 1 à 5, dans lequel la surface du support présente une couche d'un composé chimique qui empêche une fixation non spécifique de molécules biologiques à la surface du support.

7. Elément fonctionnel selon l'une quelconque des revendications 1 à 6, dans lequel le polymère utilisé comme moyen de liaison est un hydrogel.

8. Elément fonctionnel selon l'une quelconque des revendications 1 à 7, le moyen de liaison peut être relié par modification de la valeur du pH, de la concentration ionique ou de la température.

9. Elément fonctionnel selon l'une quelconque des revendications 1 à 8, dans lequel les nanoparticules comprennent un noyau et une surface présentant les points de reconnaissance spécifiques à une molécule.

10. Elément fonctionnel selon la revendication 9, dans lequel une ou plusieurs molécules biologiquement actives sont liées aux points de reconnaissance spécifiques à une molécule.

11. Elément fonctionnel selon la revendication 10, dans lequel les molécules biologiquement actives sont liées de manière covalente et/ou non covalente.

12. Elément fonctionnel selon la revendication 10 ou 11, dans lequel les molécules sont liées en conservant leur activité biologique.

13. Elément fonctionnel selon l'une quelconque des revendications 10 à 12, dans lequel il s'agit pour les molécules liées de protéines, d'acides nucléiques, de molécules de PNA ou de fragments de ceux-ci.

14. Elément fonctionnel selon la revendication 13, dans lequel les protéines sont des anticorps, des antigènes, des enzymes, des cytokines ou des récepteurs.

15. Elément fonctionnel selon l'une quelconque des revendications 1 à 14, dans lequel les premiers groupes fonctionnels et les seconds groupes fonctionnels complémentaires fixant les premiers groupes fonctionnels sont sélectionnés parmi le groupe constitué d'un ester actif, d'un groupe d'alkylcétone, d'un groupe aldéhyde, d'un groupe amino, d'un groupe carboxy, d'un groupe époxy, d'un groupe maléinimido, d'un groupe hydrazine, d'un groupe hydrazide, d'un groupe thiol, d'un groupe thioester, d'un groupe oligohistidine, de Strep-Tag I, de Strep-Tag II, de la desthiobiotine, de la biotine, de la chitine, de dérivés de chitine, d'un domaine de liaison à la chitine, d'un complexe de chélate métallique, de la streptavidine, de la streptactine, de l'avidine et de la neutravidine.

16. Elément fonctionnel selon la revendication 15, dans lequel les premiers et les seconds groupes fonctionnels sont générés par marquage moléculaire.

17. Elément fonctionnel selon la revendication 15 ou 16, dans lequel les premiers groupes fonctionnels font partie d'un espaceur ou sont reliés par le biais d'un espaceur à la surface des nanoparticules.

18. Elément fonctionnel selon la revendication 15 ou 16, dans lequel les seconds groupes fonctionnels complémentaires font partie d'un espaceur ou sont reliés par le biais d'un espaceur aux molécules.

19. Elément fonctionnel selon l'une quelconque des revendications 9 à 18, dans lequel le noyau des nanoparticules se compose d'une matière organique ou contient celle-ci.

20. Elément fonctionnel selon la revendication 19, dans lequel la matière organique est un polymère organique.

21. Elément fonctionnel selon la revendication 19 ou 20, dans lequel le polymère organique est le polypropylène, le polystyrène, le polyacrylate ou un mélange de ceux-ci.

22. Elément fonctionnel selon l'une quelconque des revendications 9 à 18, dans lequel le noyau se compose d'une matière inorganique ou contient celle-ci.

23. Elément fonctionnel selon la revendication 22, dans lequel la matière inorganique est un métal tel que Au, Ag ou Ni, du silicium, SiO₂, SiO, un silicate, Al₂O₃, SiO₂·Al₂O₃, Fe₂O₃, Ag₂O, TiO₂, ZrO₂, Zr₂O₃, Ta₂O₅, de la zéolithe, du verre, de l'oxyde d'indium-étain, de l'hydroxylapatite, un point quantique ou un mélange de ceux-ci.

24. Elément fonctionnel selon l'une quelconque des revendications 9 à 23, dans lequel le noyau présente une taille de 5 nm à 500 nm.

25. Elément fonctionnel selon l'une quelconque des revendications 9 à 24, dans lequel le noyau présente au moins une fonction supplémentaire.

26. Elément fonctionnel selon la revendication 25, dans lequel la fonction supplémentaire dans le noyau est ancrée et est un marquage fluorescent, un marquage UV/Vis, une fonction superparamagnétique, une fonction ferromagnétique et/ou un marquage radioactif.

27. Elément fonctionnel selon la revendication 25, dans lequel la surface du noyau est modifiée avec une couche organique ou inorganique contenant les premiers groupes fonctionnels qui présente un marquage fluorescent, un marquage UV/Vis, une fonction superparamagnétique, une fonction ferromagnétique et/ou un marquage radioactif.

28. Elément fonctionnel selon l'une quelconque des revendications 25 à 27, dans lequel la surface du noyau présente un composé chimique qui sert à la stabilisation stérique et/ou à empêcher une modification de conformation des molécules immobilisées et/ou à empêcher la fixation d'un autre composé biologiquement actif sur le noyau.

29. Elément fonctionnel selon la revendication 28, dans lequel le composé chimique est un polyéthylèneglycol, un oligoéthylèneglycol, le dextrane ou un mélange de ceux-ci.

30. Elément fonctionnel selon l'une quelconque des revendications précédentes, dans lequel les molécules liées présentent un marqueur.

31. Elément fonctionnel selon l'une quelconque des revendications précédentes, dans lequel d'autres molécules sont liées aux molécules liées.

32. Elément fonctionnel selon l'une quelconque des revendications précédentes, dans lequel la microstructure se compose d'une couche de nanoparticules.

33. Elément fonctionnel selon l'une quelconque des revendications précédentes, dans lequel la microstructure se compose de plusieurs couches de nanoparticules.

34. Elément fonctionnel selon l'une quelconque des revendications précédentes, dans lequel plusieurs microstructures, qui se composent de nanoparticules avec différents points de reconnaissance spécifiques à une molécule, sont disposées sur la surface du support.

35. Elément fonctionnel selon la revendication 34, dans lequel différentes molécules sont liées aux microstructures.

36. Elément fonctionnel selon l'une quelconque des revendications 1 à 35, pouvant être obtenu par application d'une ou plusieurs microstructures sur la surface du support en utilisant une imprimante matricielle à aiguilles.

37. Elément fonctionnel selon l'une quelconque des revendications 1 à 35, pouvant être obtenu par application d'une ou plusieurs microstructures sur la surface du support en utilisant un procédé lithographique.

38. Elément fonctionnel selon la revendication 37, dans lequel le procédé lithographique est la photolithographie.

39. Elément fonctionnel selon la revendication 37, dans lequel le procédé lithographique est la photolithographie à Micropen.

40. Elément fonctionnel selon l'une quelconque des revendications 1 à 35, pouvant être obtenu par application d'une ou plusieurs microstructures sur la surface du support en utilisant un procédé à jet d'encre.

41. Elément fonctionnel selon l'une quelconque des revendications 1 à 35, pouvant être obtenu par application d'une ou plusieurs microstructures en utilisant un procédé d'impression par microcontact.

42. Procédé pour la fabrication d'un élément fonctionnel selon l'une quelconque des revendications 1 à 41, dans lequel au moins une couche du moyen de liaison selon la revendication 1 et ensuite au moins une microstructure composée de nanoparticules avec des points de reconnaissance spécifiques à une molécule selon la revendication 1 sont appliquées sur la surface d'un support.

43. Procédé selon la revendication 42, dans lequel la surface du support est nettoyée et/ou activée avant l'application de la couche de moyen de liaison.

44. Procédé selon la revendication 43, dans lequel la surface du support est activée par voie chimique.

45. Procédé selon la revendication 44, dans lequel la surface du support est pourvue de charges.

46. Procédé selon la revendication 44 ou 45, dans lequel la surface du support est activée par application d'une amorce.

47. Procédé selon la revendication 44 ou 45, dans lequel une couche d'autoassemblage est appliquée sur la surface du support.

48. Procédé selon la revendication 43, dans lequel la surface du support est activée au moyen d'un plasma.

49. Procédé selon l'une quelconque des revendications 42 à 48, dans lequel une couche de moyen de liaison définie par rapport à la forme et la surface est appliquée sur la surface du support et le support est ensuite immergé dans une suspension de nanoparticules de sorte qu'une microstructure définie par rapport à la forme et la surface est produite par adhésion des nanoparticules à la couche de moyen de liaison appliquée.

50. Procédé selon la revendication 49, dans lequel la couche de moyen de liaison définie par rapport à la forme et la surface est appliquée au moyen d'une imprimante matricielle à aiguilles, d'un procédé lithographique, d'un procédé à jet d'encre ou d'un procédé d'impression par microcontact.

51. Procédé selon l'une quelconque des revendications 42 à 50, dans lequel le support est immergé dans une suspension ou solution du moyen de liaison de sorte qu'une couche de moyen de liaison recouvrant l'ensemble de la surface du support est produite, et les nanoparticules sont ensuite appliquées de sorte qu'une microstructure définie par rapport à la forme et la surface est produite.

52. Procédé selon la revendication 51, dans lequel la microstructure définie par rapport à la forme et la surface est appliquée au moyen d'une imprimante matricielle à aiguilles, d'un procédé lithographique, d'un procédé à jet d'encre ou d'un procédé d'impression par microcontact.

53. Procédé selon l'une quelconque des revendications 42 à 52, dans lequel le moyen de liaison et les nanoparticules sont appliqués plusieurs fois sur la surface du support.

54. Procédé selon l'une quelconque des revendications 42 à 53, dans lequel des molécules biologiquement actives sont liées aux points de reconnaissance spécifiques à une molécule des nanoparticules avant application des nanoparticules.

55. Procédé selon l'une quelconque des revendications 42 à 53, dans lequel des molécules biologiquement actives sont liées aux points de reconnaissance spécifiques à une molécule des nanoparticules après application des nanoparticules.

56. Procédé selon l'une quelconque des revendications 42 à 53, dans lequel des molécules biologiquement actives sont liées aux points de reconnaissance spécifiques à une molécule des nanoparticules avant et après application des nanoparticules.

57. Procédé selon l'une quelconque des revendications 54 à 56, dans lequel la liaison des molécules biologiquement actives aux points de reconnaissance spécifiques à une molécule des nanoparticules s'effectue en ce que les points de reconnaissance spécifiques à une molécule des nanoparticules, présentant des premiers groupes fonctionnels, sont amenés en contact avec les molécules présentant des seconds groupes fonctionnels complémentaires, fixant les premiers groupes fonctionnels, de telle sorte que des liaisons covalentes et/ou non covalentes ont lieu entre les groupes fonctionnels des points de reconnaissance spécifiques à une molécule et les molécules.

58. Procédé selon la revendication 57, dans lequel les premiers groupes fonctionnels et les seconds groupes fonctionnels complémentaires fixant les premiers groupes fonctionnels sont sélectionnés parmi le groupe constitué d'un ester actif, d'un groupe d'alkylcétone, d'un groupe aldéhyde, d'un groupe amino, d'un groupe carboxy, d'un groupe époxy, d'un groupe maléinimido, d'un groupe hydrazine, d'un groupe hydrazide, d'un groupe thiol, d'un groupe thioester, d'un groupe oligohistidine, de Strep-Tag I, de Strep-Tag II, de la desthiobiotine, de la biotine, de la chitine, de dérivés de chitine, d'un domaine de liaison à la chitine, d'un complexe de chélate métallique, de la streptavidine, de la streptactine, de l'avidine et de la neutravidine.

59. Procédé selon l'une quelconque des revendications 54 à 58, dans lequel les molécules biologiquement actives sont liées en conservant leur activité biologique.

60. Procédé selon l'une quelconque des revendications 54 à 59, dans lequel il s'agit pour les molécules de protéines, d'antigènes, d'acides nucléiques, de molécules de PNA ou de fragments de ceux-ci.

61. Utilisation d'un élément fonctionnel selon l'une quelconque des revendications 1 à 41 ou d'un élément fonctionnel fabriqué selon un procédé selon l'une quelconque des revendications 42 à 60 en vue de réaliser un procédé de détection.

62. Utilisation selon la revendication 61, dans laquelle le procédé de détection est une spectroscopie de masse MALDI, une spectroscopie de fluorescence ou UV-Vis, une microscopie en fluorescence ou optique, une spectroscopie à guide d'onde, la spectroscopie d'impédance ou un autre procédé électrique.

63. Utilisation d'un élément fonctionnel selon l'une quelconque des revendications 1 à 41 ou d'un élément fonctionnel fabriqué selon un procédé selon l'une quelconque des revendications 42 à 60 en vue de commander l'adhésion cellulaire ou la croissance cellulaire.

64. Utilisation d'un élément fonctionnel selon l'une quelconque des revendications 1 à 41 ou d'un élément fonctionnel fabriqué selon un procédé selon l'une quelconque des revendications 42 à 60 en vue de développer des préparations pharmaceutiques.

65. Utilisation d'un élément fonctionnel selon l'une quelconque des revendications 1 à 41 ou d'un élément fonctionnel fabriqué selon un procédé selon l'une quelconque des revendications 42 à 60 en vue de l'analyser des effets et/ou effets secondaires de préparations pharmaceutiques.

66. Utilisation d'un élément fonctionnel selon l'une quelconque des revendications 1 à 41 ou d'un élément fonctionnel fabriqué selon un procédé selon l'une quelconque des revendications 42 à 60 en vue du diagnostic de maladies.

67. Utilisation selon la revendication 66, dans laquelle l'élément fonctionnel est utilisé pour l'identification d'agents pathogènes.

68. Utilisation selon la revendication 66, dans laquelle l'élément fonctionnel est utilisé pour l'identification de gènes mutés chez l'homme ou l'animal.

69. Utilisation d'un élément fonctionnel selon l'une quelconque des revendications 1 à 41 ou d'un élément fonctionnel fabriqué selon un procédé selon l'une quelconque des revendications 42 à 60 en vue de l'analyse de la contamination microbiologique d'échantillons.

70. Utilisation selon la revendication 69, dans laquelle l'échantillon est un échantillon d'eau ou de sol.

71. Utilisation selon la revendication 69, dans laquelle l'échantillon provient d'un aliment ou d'aliments pour animaux.

72. Utilisation d'un élément fonctionnel selon l'une quelconque des revendications 1 à 41 ou d'un élément fonctionnel fabriqué selon un procédé selon l'une quelconque des revendications 42 à 60 en tant que composant électronique dans un bio-ordinateur.
